# EUROPEAN PATENT APPLICATION

(11) **EP 4 553 082 A2**
(43) Date of publication of application: **14.05.2025**
(21) Application number: 25166580.8
(22) Date of filing: 03.11.2017
(51) Int. Cl.: C07J 51/00

(54) **LIVER ORGANOID COMPOSITIONS AND METHODS OF MAKING AND USING SAME**

(30) Priority: 04.11.2016 US 201662417371 P; 09.06.2017 US 201762517414 P
(62) Divisional of application: 17867910.6
(71) Applicant: Children's Hospital Medical Center, Cincinnati, Ohio 45229-3029 (US); Japan Science and Technology Agency, Kawaguchi-shi, Saitama 332-0012 (JP)
(72) Inventor: TAKEBE,, Takanori, Cincinnati, 45229 (US); SHINOZAWA,, Tadahiro, Cincinnati, 45229 (US); KIMURA,, Masaki, Cincinnati, 45229 (US); KOIKE,, Hiroyuki, Cincinnati, 45229 (US)
(74) Representative: Potter Clarkson

(57) **Abstract**

Disclosed are methods of inducing formation of a liver organoid from precursor cells, such as iPSC cells. The disclosed liver organoids may be used for screening for a serious adverse event (SAE), such as liver failure and/or drug induced liver injury (DILI), and/or drug toxicity. The disclosed liver organoids may also be used to treat an individual having liver damage, or for identifying a preferred therapeutic agent.

## Description

### Cross Reference to Related Applications

This application claims priority to and benefit of U.S. Provisional Patent Application 62/471,371, filed November 4, 2016, and 62/517,414, filed June 9, 2016, the contents of each are incorporated by reference in their entirety for all purposes.

### Background

The liver is a vital organ that provides many essential metabolic functions for life such as the detoxification of exogenous compounds and coagulation as well as producing lipids, proteins, ammonium, and bile. In vitro reconstitution of a patient's liver may provide applications including regenerative therapy, drug discovery and drug toxicity studies. Existing methodology using liver cells exhibit extremely poor functionality, largely due to a lack of essential anatomical structures, which limits their practical use for the pharmaceutical industry.

Billions of dollars are lost annually from drug development in the pharmaceutical industry due to the failures of drug candidates identified in initial screens, and nearly a third of drugs are withdrawn from the market due to such failures (Takebe and Taniguchi, 2014). A failure of drug candidates results in a tremendous loss of a patient's treatment opportunity. Preclinical studies generally consist of an *in vitro* evaluation as a primary efficacy screen to identify a "hit" compound, followed by safety studies *in vitro* and *in vivo* to assess the mechanisms of metabolism and toxicology. This inefficiency can be explained by the substantial lack of physiologically relevant preclinical models with high throughput in evaluating drug induced liver injury (DILI) in humans and thus, an urgent need to develop an in vitro humanistic screen model for the evaluation of the vast amounts of continuously growing compound libraries.

Primary hepatocytes are a highly polarized metabolic cell type, and form a bile canaliculi structure with microvilli-lined channels, separating peripheral circulation from the bile acid secretion pathway. The most upstream aspects of DILI include drug (or their reactive metabolites) detoxification by hepatocytes and excretion into bile canaliculi through transporters such as multi-drug resistance-associated protein (MRP) transporters. This suggests the need to reconstruct these uniquely organized structures as a crucial property of hepatocytes *in vivo* for predicting DILI pathology. However, there are considerable differences in drug toxicity profiles between the current simplified culture model with the use of isolated primary human hepatocytes or hepatic cell lines, and *in vivo* physiology, resulting in failed translation of drugs or drug discontinuation, as in the case of Troglitazone, Nefazodone and Tolcapone (https://livertox.nlm.nih.gov/index.html). The determination of toxicological properties thus mainly relies on animals as an essential step for drug development, however, due to the pronounced differences in physiology between humans and animals, there is a significant lack of fidelity to human outcomes (Leslie et al., 2007; Yang et al., 2014). In addition, the onset of idiosyncratic DILI (IDILI), which is very rare but nonetheless responsible for about 10-15% of acute liver failures in the USA (Reuben et al., 2010), is almost impossible to predict (Kullak-Ublick et al., 2017). Collectively, effective human cell models are needed to screen for compounds that test the detoxification and excretion of proposed drugs.

Despite the progressive advancement of human hepatocyte differentiation methods from pluripotent stem cell (PSC), clinical trials in a dish using human stem cells remain 'hype'.[Besides drug screens for efficacy and/or toxicity, there is a need for liver cell models for use in bio-artificial liver devices as a bridge for transplant, for example, and for precision (personalized medicine). The instant disclosure seeks to address one or more of the aforementioned needs in the art.

### Brief Summary

Disclosed are methods of inducing formation of a liver organoid from precursor cells, such as iPSC cells. The disclosed liver organoids may be used for screening for a serious adverse event (SAE), such as liver failure and/or drug induced liver injury (DILI), and/or drug toxicity. The disclosed liver organoids may also be used to treat an individual having liver damage, or for identifying a preferred therapeutic agent.

### Brief Description of the Drawings

Those of skill in the art will understand that the drawings, described below, are for illustrative purposes only. The drawings are not intended to limit the scope of the present teachings in any way.

The patent or application file contains at least one drawing executed in color. Copies of this patent or patent application publication with color drawing(s) will be provided by the Office upon request and payment of the necessary fee.

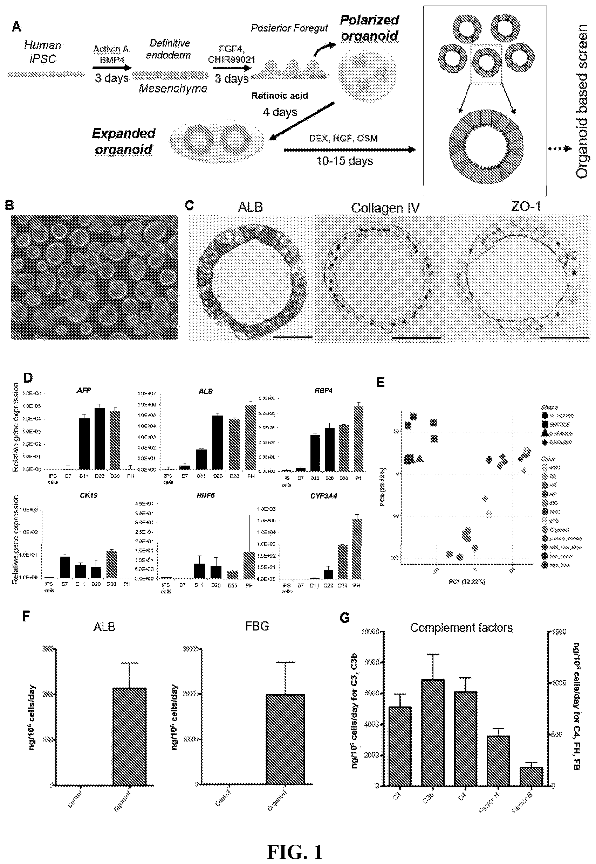

FIG 1. Generation of human liver organoid from iPSC with luminal structure. A. Overview of the differentiation method for liver organoid. B. Phase contrast image of human liver organoids. C. Immunostaining for Albumin (ALB), Type IV collagen (Collagen IV) and ZO-1 in organoids. Nuclei were stained with Haematoxylin (blue). Bars, 50 µm. D. Quantitative RT-PCR of Alpha-fetoprotein (AFP), Albumin (ALB), Retinol-Binding Protein 4 (RBP4), Cytokeratin 19 (CK19), Hepatocyte nuclear factor 6 (HNF6) and Cytochrome P450 3A4 (CYP3A4) in undifferentiated iPS cells, organoids at Day 7, 11, 20 and 30 of differentiation and Primary hepatocytes (PH). Relative expression values were compared with undifferentiated iPSCs (AFP, ALB, RBP4 and CK19) or Day 7 organoids (HNF6) or Day 11 organoids (CYP3A4). Bars represent the mean ± SD, n = 3. E. Principal component analysis based on RNA sequence data in undifferentiated iPS cells (iPSC), Definitive endoderm (DE), Hepatic Specified cells (HS), Hepatic Progenitor (HP), iPSC-Derived Cholangiocytes (iDC), Normal Human Cholangiocyte (NHC), iPSC-derived Posterior foregut (pFG), iPSC-derived human liver organoid , Primary hepatocytes, Fetal liver tissue, Liver tissue and Right lobe of human Liver. F. Albumin (ALB, n = 10) and Fibrinogen (FBG, n = 4) secretion levels from organoids at Day 25-30. Bars represent the mean ± SEM. G. Complement factors secretion level from organoids at Day 25-30. FH: Factor H, FB: Factor B. Bars represent the mean ± SEM, n = 5.

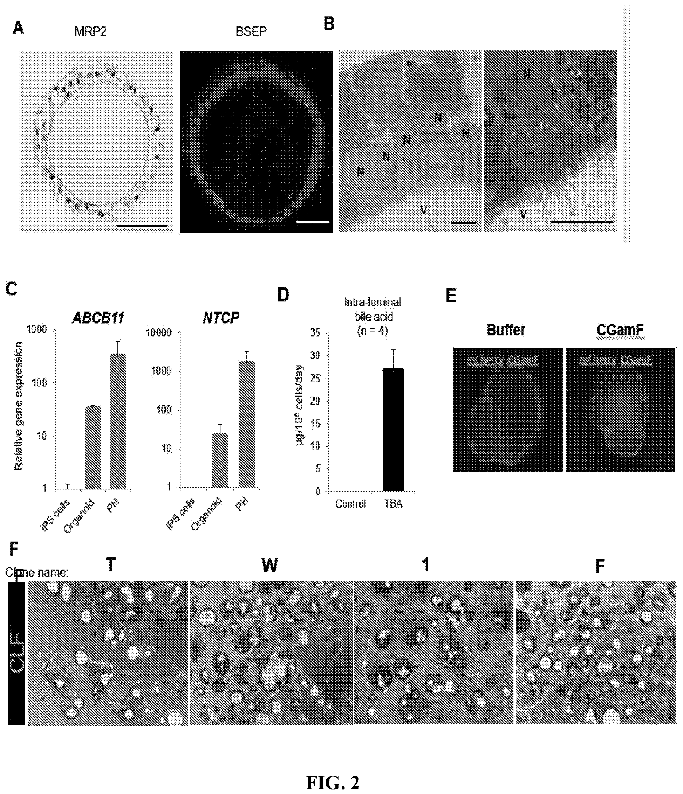

FIG 2. Bile acid synthesis, uptake and excretion in human iPSC liver organoid. A. Immunostaining for Multidrug resistance-associated protein 2 (MRP2) and Bile salt export pump (BSEP) in a single organoid. Bars, 50 µm. B. Transmission electron micrograph of organoid showing microvilli (V) intra-luminal surface; N: nuclei. Bars, 10 µm. C. Quantitative RT-PCR of ATP-binding cassette, sub-family B member 11 (ABCB11) and Sodium taurocholate co-transporting polypeptide (NTCP) in undifferentiated iPSCs, organoids at Day 20 (NTCP) and 30 (ABCB11) of differentiation and Primary hepatocytes (PH). Relative expression value was compared with undifferentiated iPS cells (ABCB11) or Day 11 organoids (NTCP). Bars represent the mean ± SD, n = 3. D. Total bile acid secretion level inside organoids at day 27. Bars represent the mean ± SEM, n=4. E. Bile acid uptake by organoid after 30 min of culture in the presence of fluorescent bile acid (CGamF). F. CLF transport activity on organoids derived from 4 iPSC lines. T, W, 1 and F indicate clone name of iPS cell lines. Green: CLF.

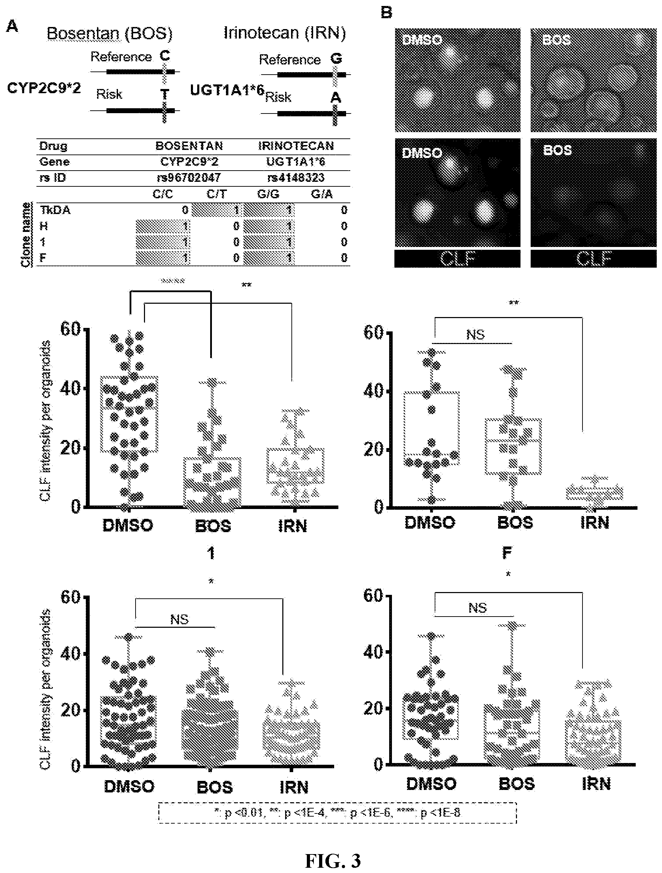

**FIG** 3. Bosetan induced cholestasis is specific to CYP2C9*2 iPSC-liver organoids. A. Representative allele images of rs1799853 in CYP2C9*2 and rs4148323 in UGT1A1*6 show risk SNPs for DILI by Bosentan and Irinotecan, respectively. The table indicates the possession of risk alleles in each iPS cell line. B. Images of CLF transport activity and inhibition by Bosentan. C. CLF intensity levels in individual organoids derived from different 4 iPS cell lines. *: p <0.01, **: p <1E-4, ****: p <1E-8, Wilcoxon-Mann-Whitney Test. NS: not significant. In the box plots, the top and bottom of the box represent the 75th and 25th percentiles, the center line represents the median. Dot indicates the data from each organoid.

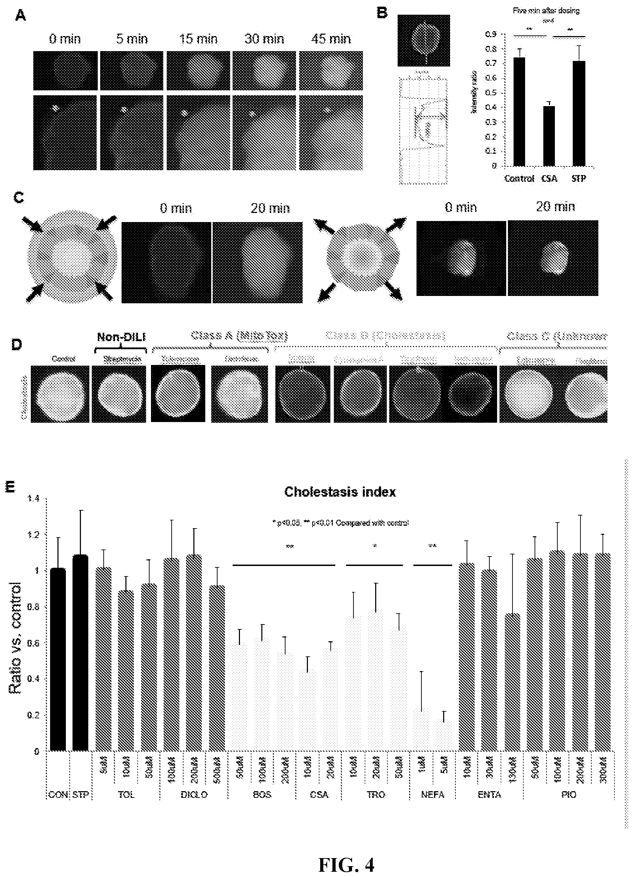

FIG 4. High fidelity drug induced cholestasis model using organoids. A. Sequential images for efflux of fluorescein diacetate from outside to inside of the organoids. B. Comparison of fluorescein diacetate efflux transport. C. Quantification of fluorescein diacetate efflux transport into organoid. Example left image was quantified ratio of fluorescein intensity between inside and outside the organoid. Right graph indicates the result of validation study using control (DMSO), Cyclosporin A (CSA) and Streptomycin (STP) as negative control. Bars represent the mean ± SD, **: p < 0.01, n = 4. D. Image of fluorescein diacetate transport inhibition after treatment of 9 training compounds for 24h. E. Quantification of transport inhibition after treatment of training compounds, Bars represent the mean ± SD, *: p<0.05, **: p<0.01, n= 4-6. Quantification of MMP change after treatment of training compounds, Bars represent the mean ± SD, *: p<0.05, **: p<0.01, n= 4-5. CON: Control sample, STP: Streptomycin, TOL: Tolcapone, DICLO: Diclofenac, BOS: Bosentan, CSA: Cyclosporin A.

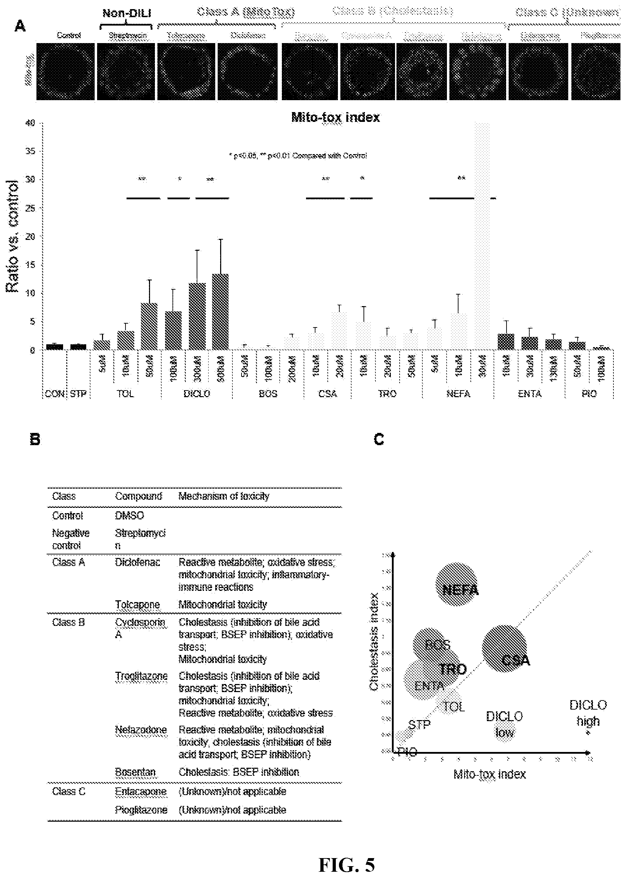

FIG 5. High-fidelity drug induced mitochondria-toxicity screen using organoids. A. Image of mitochondria membrane potential (MMP) on TMRM after treatment of 9 training compounds. Lower: Quantification of transport inhibition after treatment of training compounds, Bars represent the mean ± SD, *: p<0.05, **: p<0.01, n= 4-6. C. Quantification of MMP change after treatment of training compounds, Bars represent the mean ± SD, *: p<0.05, **: p<0.01, n= 4-5. CON: Control sample, STP: Streptomycin, TOL: Tolcapone, DICLO: Diclofenac, BOS: Bosentan, CSA: Cyclosporin A, TRO: Troglitazone, NEFA: Nefazodone, ENTA: Entacapone, PIO: Pioglitazone. B. Classification of the set of 9 training compounds (TC) referred to Oorts., et al 2016 (Oorts et al., 2016). Class A represents TCs with known reports on DILI in vivo, while those in Class B are TCs with reports on drug-induced cholestasis in vivo. The mechanism of toxicity based on literature data is also provided. Class C compounds are generally considered safe regarding DILI. C. Analysis between viability for 72h after treatment of drugs and dual risk parameters, drug-induced cholestasis potential and mitochondria toxicity potential. Cholestasis and Mitochondria toxicity (Mito-tox) indexes were derived from data in Figure 3. The size of circles indicated the magnitude of viability decreases.

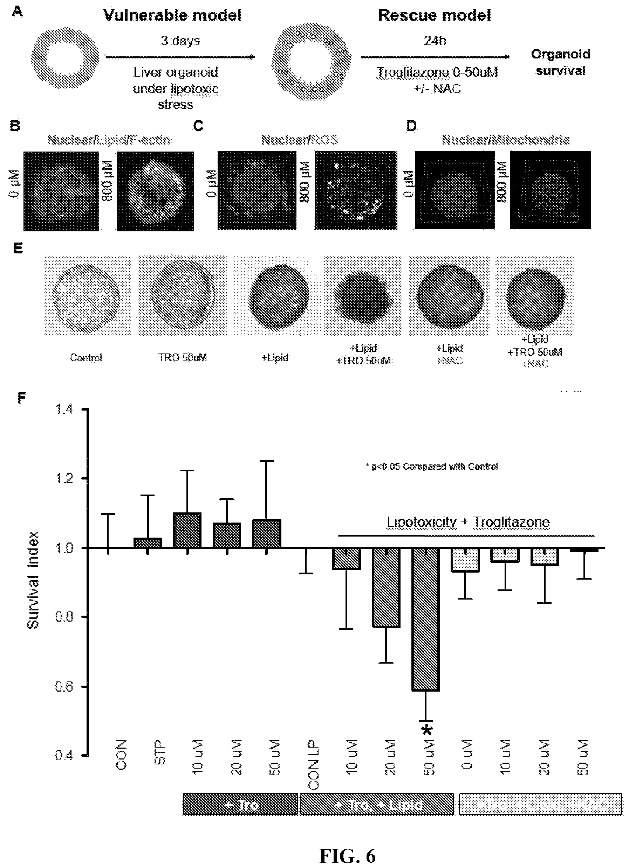

FIG 6. Modeling drug-induced liver injury in vulnerable conditions rescued by NAC exposure. A. Overview of evaluation of drug-induced cytotoxicity on vulnerable organoid model. B. Profiling of vulnerable model on lipid accumulation (Blue: nuclei, Green: Lipid, Red: F-actin). C ROS production (Blue: nuclei, Green: ROS) and D. mitochondria health (Blue: nuclei, Red: Mitochondria). E. Image of organoids at 24h after drugs treatment. F. Viability assessment on lipid accumulation-induced vulnerable organoid model. Bars represent the mean ± SD, *: p<0.05, n= 5-6. CON: control, STP: Streptomycin, TRO: Troglitazone, NAC: N-acetylcysteine.

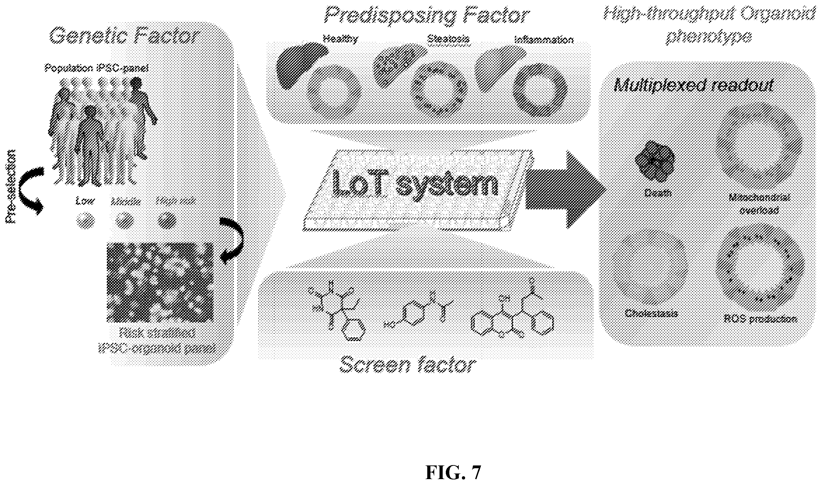

FIG 7. Multiplexed liver organoid based screening for predicting toxicity

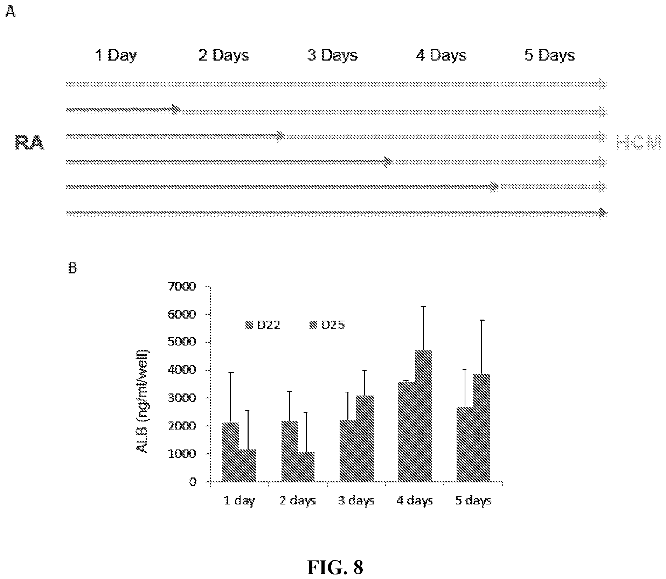

FIG 8. Optimization of retinoic acid treatment protocols A. Scheme for timing and duration of retinoic acid treatment. RA: retinoic acid, HCM: hepatocyte culture medium. B. Albumin secretion level in organoids at day 25 in different duration of RA treatment.

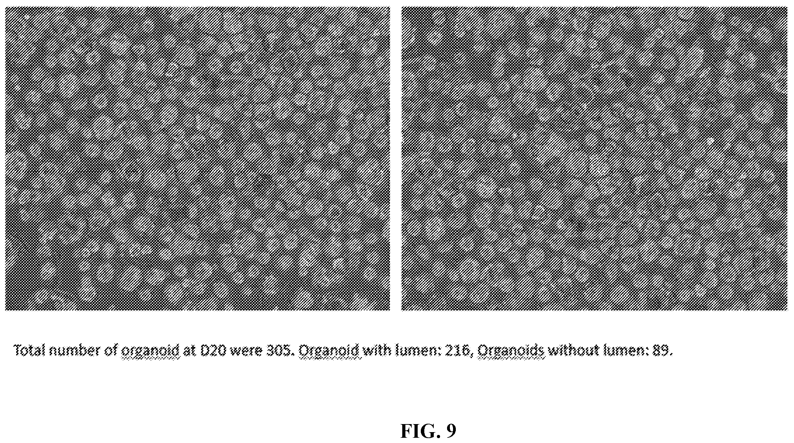

FIG 9. The morphology of organoids at D20 Total number of organoid at D20 were 305. Organoid with lumen: 216, Organoids without lumen: 89.

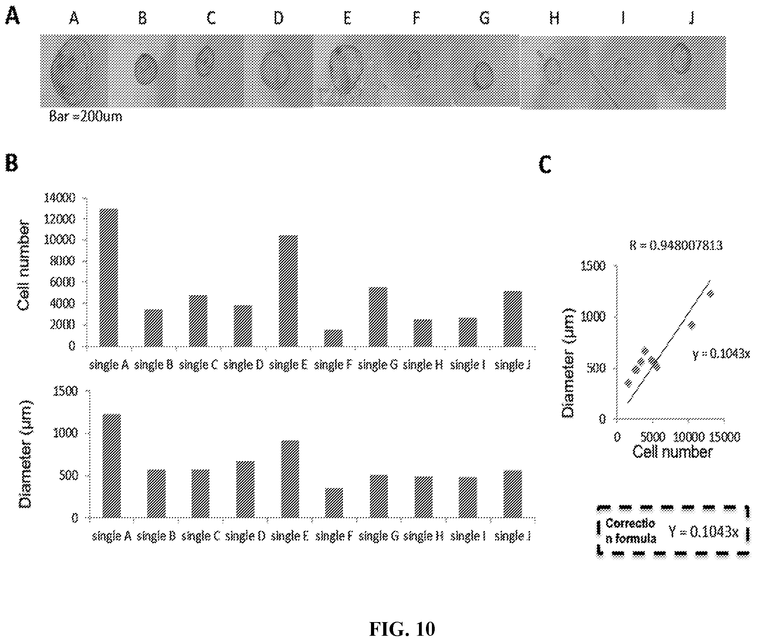

FIG 10. Conversion formula to determine the number of cells in organoids A. Phase contrast image of single organoids. B. The diameter and cell number of each single organoid. C. Correlation between diameter and cell number in single organoid.

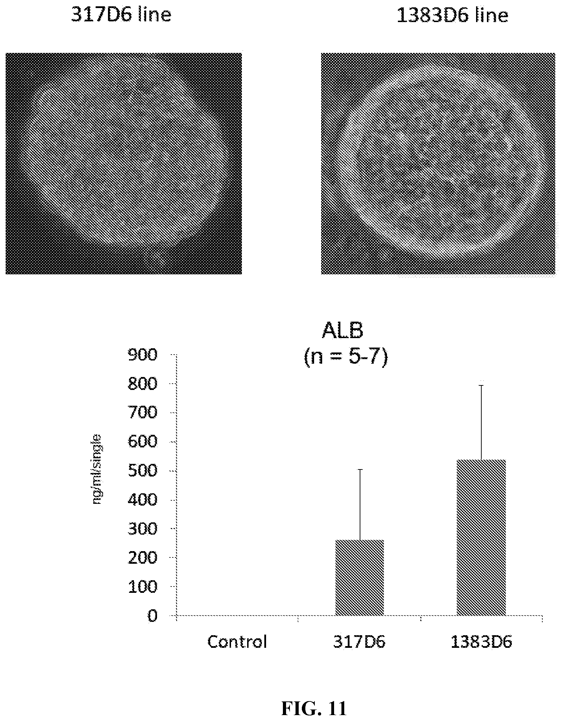

FIG 11 - Supplementary FIG 4 The generation of organoids from multiple PSC lines. Phase contrast image and albumin secretion level of different iPS cell line (317D6 and 1383D6)-derived organoid.

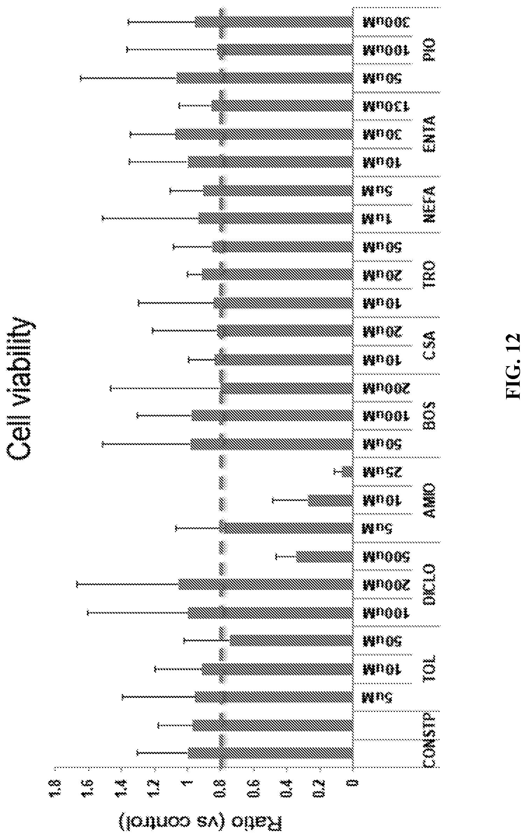

FIG 12. The cell viability at 24h after treatment of 10 compounds. Viability assessment on lipid accumulation-induced vulnerable organoid model. CON: Control sample, STP: Streptomycin, TOL: Tolcapone, DICLO: Diclofenac, AMIO: Amiodarone, BOS: Bosentan, CSA: Cyclosporin A, TRO: Troglitazone, NEFA: Nefazodone, ENTA: Entacapone, PIO: Pioglitazone. Bars represent the mean ± SD, n= 4-6.

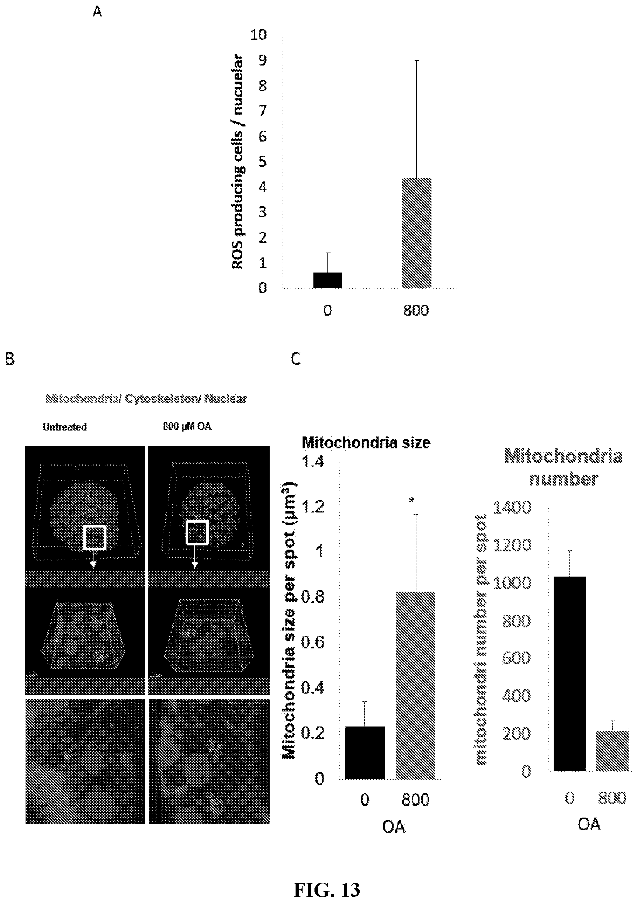

FIG 13. ROS production and the morphological change of mitochondria in lipotoxic liver organoid. A. The ratio of cell number producing ROS in total cells on lipid accumulation-induced vulnerable organoid model by treatment of 800 µM oleic acid (OA). B. Image of mitochondria in organoid on vulnerable organoid model. Red: mitochondria, Purple: F-actin, Blue: Nucleus. C. The number and size of mitochondria on vulnerable organoid model. Bars represent the mean ± SD, *: p<0.05, n= 5-6.

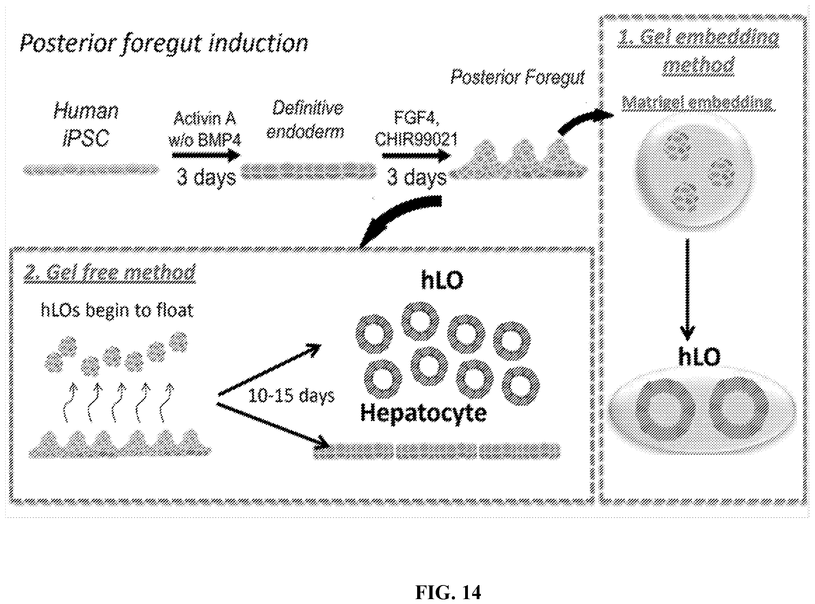

FIG 14. Schematic of Cell Matrigel-Free Method. Shown is a schematic for a liver organoid generation method that does not use matrigel for generating organoids.

### Detailed Description of the Invention

Unless otherwise noted, terms are to be understood according to conventional usage by those of ordinary skill in the relevant art.

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the art. Alternatively, "about" can mean a range of up to 20%, or up to 10%, or up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the term "totipotent stem cells" (also known as omnipotent stem cells) are stem cells that can differentiate into embryonic and extra-embryonic cell types. Such cells can construct a complete, viable organism. These cells are produced from the fusion of an egg and sperm cell. Cells produced by the first few divisions of the fertilized egg are also totipotent.

As used herein, the term "pluripotent stem cells (PSCs)" encompasses any cells that can differentiate into nearly all cell types of the body, i.e., cells derived from any of the three germ layers (germinal epithelium), including endoderm (interior stomach lining, gastrointestinal tract, the lungs), mesoderm (muscle, bone, blood, urogenital), and ectoderm (epidermal tissues and nervous system). PSCs can be the descendants of inner cell mass cells of the preimplantation blastocyst or obtained through induction of a non-pluripotent cell, such as an adult somatic cell, by forcing the expression of certain genes. Pluripotent stem cells can be derived from any suitable source. Examples of sources of pluripotent stem cells include mammalian sources, including human, rodent, porcine, and bovine.

As used herein, the term "induced pluripotent stem cells (iPSCs)," also commonly abbreviated as iPS cells, refers to a type of pluripotent stem cells artificially derived from a normally non-pluripotent cell, such as an adult somatic cell, by inducing a "forced" expression of certain genes. hiPSC refers to human iPSCs.

As used herein, the term "embryonic stem cells (ESCs)," also commonly abbreviated as ES cells, refers to cells that are pluripotent and derived from the inner cell mass of the blastocyst, an early-stage embryo. For purpose of the present invention, the term "ESCs" is used broadly sometimes to encompass the embryonic germ cells as well.

As used herein, the term "precursor cell" encompasses any cells that can be used in methods described herein, through which one or more precursor cells acquire the ability to renew itself or differentiate into one or more specialized cell types. In some embodiments, a precursor cell is pluripotent or has the capacity to becoming pluripotent. In some embodiments, the precursor cells are subjected to the treatment of external factors (e.g., growth factors) to acquire pluripotency. In some embodiments, a precursor cell can be a totipotent (or omnipotent) stem cell; a pluripotent stem cell (induced or non-induced); a multipotent stem cell; an oligopotent stem cells and a unipotent stem cell. In some embodiments, a precursor cell can be from an embryo, an infant, a child, or an adult. In some embodiments, a precursor cell can be a somatic cell subject to treatment such that pluripotency is conferred via genetic manipulation or protein/peptide treatment.

In developmental biology, cellular differentiation is the process by which a less specialized cell becomes a more specialized cell type. As used herein, the term "directed differentiation" describes a process through which a less specialized cell becomes a particular specialized target cell type. The particularity of the specialized target cell type can be determined by any applicable methods that can be used to define or alter the destiny of the initial cell. Exemplary methods include but are not limited to genetic manipulation, chemical treatment, protein treatment, and nucleic acid treatment.

### Pluripotent Stem Cells Derived from Embryonic Cells

In some embodiments, one step is to obtain stem cells that are pluripotent or can be induced to become pluripotent. In some embodiments, pluripotent stem cells are derived from embryonic stem cells, which are in turn derived from totipotent cells of the early mammalian embryo and are capable of unlimited, undifferentiated proliferation in vitro. Embryonic stem cells are pluripotent stem cells derived from the inner cell mass of the blastocyst, an early-stage embryo. Methods for deriving embryonic stem cells from blastocytes are well known in the art. Human embryonic stem cells H9 (H9-hESCs) are used in the exemplary embodiments described in the present application, but it would be understood by one of skill in the art that the methods and systems described herein are applicable to any stem cells.

Additional stem cells that can be used in embodiments in accordance with the present invention include but are not limited to those provided by or described in the database hosted by the National Stem Cell Bank (NSCB), Human Embryonic Stem Cell Research Center at the University of California, San Francisco (UCSF); WISC cell Bank at the Wi Cell Research Institute; the University of Wisconsin Stem Cell and Regenerative Medicine Center (UW-SCRMC); Novocell, Inc. (San Diego, Calif.); Cellartis AB (Goteborg, Sweden); ES Cell International Pte Ltd (Singapore); Technion at the Israel Institute of Technology (Haifa, Israel); and the Stem Cell Database hosted by Princeton University and the University of Pennsylvania. Exemplary embryonic stem cells that can be used in embodiments in accordance with the present invention include but are not limited to SA01 (SA001); SA02 (SA002); ES01 (HES-1); ES02 (HES-2); ES03 (HES-3); ES04 (HES-4); ES05 (HES-5); ES06 (HES-6); BG01 (BGN-01); BG02 (BGN-02); BG03 (BGN-03); TE03 (13); TE04 (14); TE06 (16); UC01 (HSF1); UC06 (HSF6); WA01 (H1); WA07 (H7); WA09 (H9); WA13 (H13); WA14 (H14).

More details on embryonic stem cells can be found in, for example, Thomson et al., 1998, "Embryonic Stem Cell Lines Derived from Human Blastocysts," Science 282 (5391):1145-1147; Andrews et al., 2005, "Embryonic stem (ES) cells and embryonal carcinoma (EC) cells: opposite sides of the same coin," Biochem Soc Trans 33:1526-1530; Martin 1980, "Teratocarcinomas and mammalian embryogenesis,". Science 209 (4458):768-776; Evans and Kaufman, 1981, "Establishment in culture of pluripotent cells from mouse embryos," Nature 292(5819): 154-156; Hlimanskaya et al., 2005, "Human embryonic stem cells derived without feeder cells," Lancet 365 (9471): 1636-1641; each of which is hereby incorporated herein in its entirety.

### Induced Pluripotent Stem Cells (iPSCs)

In some embodiments, iPSCs are derived by transfection of certain stem cell-associated genes into non-pluripotent cells, such as adult fibroblasts. Transfection is typically achieved through viral vectors, such as retroviruses. Transfected genes include the master transcriptional regulators Oct-3/4 (Pouf51) and Sox2, although it is suggested that other genes enhance the efficiency of induction. After 3-4 weeks, small numbers of transfected cells begin to become morphologically and biochemically similar to pluripotent stem cells, and are typically isolated through morphological selection, doubling time, or through a reporter gene and antibiotic selection. As used herein, iPSCs include but are not limited to first generation iPSCs, second generation iPSCs in mice, and human induced pluripotent stem cells. In some embodiments, a retroviral system is used to transform human fibroblasts intopluripotent stem cells using four pivotal genes: Oct3/4, Sox2, Klf4, and c-Myc. In alternative embodiments, a lentiviral system is used to transform somatic cells with OCT4, SOX2, NANOG, and LIN28. Genes whose expression are induced in iPSCs include but are not limited to Oct-3/4 (e.g., Pou5fl); certain members of the Sox gene family (e.g., Sox1, Sox2, Sox3, and Sox15); certain members of the Klf family (e.g., Klf1, Klf2, Klf4, and Klf5), certain members of the Myc family (e.g., C-myc, L-myc, and N-myc), Nanog, and LIN28.

In some embodiments, non-viral based technologies are employed to generate iPSCs. In some embodiments, an adenovirus can be used to transport the requisite four genes into the DNA of skin and liver cells of mice, resulting in cells identical to embryonic stem cells. Since the adenovirus does not combine any of its own genes with the targeted host, the danger of creating tumors is eliminated. In some embodiments, reprogramming can be accomplished via plasmid without any virus transfection system at all, although at very low efficiencies. In other embodiments, direct delivery of proteins is used to generate iPSCs, thus eliminating the need for viruses or genetic modification. In some embodiment, generation of mouse iPSCs is possible using a similar methodology: a repeated treatment of the cells with certain proteins channeled into the cells via poly-arginine anchors was sufficient to induce pluripotency. In some embodiments, the expression of pluripotency induction genes can also be increased by treating somatic cellswith FGF2 under low oxygen conditions.

More details on embryonic stem cells can be found in, for example, Kaji et al., 2009, "Virus free induction of pluripotency and subsequent excision of reprogramming factors," Nature 458:771-775; Woltjen et al., 2009, "piggyBac transposition reprograms fibroblasts to induced pluripotent stem cells," Nature 458:766-770; Okita et al., 2008, "Generation of Mouse Induced Pluripotent Stem Cells Without Viral Vectors," Science 322(5903):949-953; Stadtfeld et al., 2008, "Induced Pluripotent Stem Cells Generated without Viral Integration," Science 322(5903):945-949; and Zhou et al., 2009, "Generation of Induced Pluripotent Stem Cells Using Recombinant Proteins," Cell Stem Cell 4(5):381-384; each of which is hereby incorporated herein in its entirety.

In some embodiments, exemplary iPS cell lines include but not limited to iPS-DF19-9; iPS-DF19-9; iPS-DF4-3; iPS-DF6-9; iPS(Foreskin); iPS(IMR90); and iPS(IMR90).

More details on the functions of signaling pathways relating to DE development can be found in, for example, Zorn and Wells, 2009, "Vertebrate endoderm development and organ formation," Annu Rev Cell Dev Biol 25:221-251; Dessimoz et al., 2006, "FGF signaling is necessary for establishing gut tube domains along the anterior-posterior axis in vivo," Mech Dev 123:42-55; McLin et al., 2007, "Repression of Wnt/β-catenin signaling in the anterior endoderm is essential for liver and pancreas development. Development," 134:2207-2217; Wells and Melton, 2000, Development 127:1563-1572; de Santa Barbara et al., 2003, "Development and differentiation of the intestinal epithelium," Cell Mol Life Sci 60(7): 1322-1332; each of which is hereby incorporated herein in its entirety.

Any methods for producing definitive endoderm from pluripotent cells (e.g., iPSCs or ESCs) are applicable to the methods described herein. Any method for producing definitive endoderm from pluripotent cells (e.g., iPSCs or ESCs) are applicable to the methods described herein. Exemplary methods are disclosed in, for example, "Methods and systems for converting precursor cells into intestinal tissues through directed differentiation," US9719068B2 to Wells et al., and "Methods and systems for converting precursor cells into gastric tissues through directed differentiation," US20170240866A1, to Wells et al. In some embodiments, pluripotent cells are derived from a morula. In some embodiments, pluripotent stem cells are stem cells. Stem cells used in these methods can include, but are not limited to, embryonic stem cells. Embryonic stem cells can be derived from the embryonic inner cell mass or from the embryonic gonadal ridges. Embryonic stem cells or germ cells can originate from a variety of animal species including, but not limited to, various mammalian species including humans. In some embodiments, human embryonic stem cells are used to produce definitive endoderm. In some embodiments, human embryonic germ cells are used to produce definitive endoderm. In some embodiments, iPSCs are used to produce definitive endoderm. Additional methods for obtaining or creating DE cells that can be used in the present invention include but are not limited to those described in United States Patent No. 7,510,876 to D'Amour et al.; United States Patent No. 7,326,572 to Fisk et al.; Kubo1 et al., 2004, "Development of definitive endoderm from embryonic stem cells in culture," Development 131:1651-1662; D' Amour et al., 2005, "Efficient differentiation of human embryonic stem cells to definitive endoderm," Nature Biotechnology 23:1534-1541; and Ang et al., 1993, "The formation and maintenance of the definitive endoderm lineage in the mouse: involvement of HNF3/forkhead proteins," Development 119:1301-1315.

Applicant has discovered methods for producing 3D liver structures using human iPSCs. The structures comprise micro-liver architectures, including polarized hepatic epithelium, stellate cells, and canalicula structures. The disclosed compositions display improvements in hepatic functions, bile transport activity, and durability compared to existing models. The 3D structures model may be used as a new and robust model for drug screening tests and/or drug toxicity screening, transplantation, production of serum protein products, and development of personalized therapy. In one particular application, the compositions and methods may be used to screen drug compounds for liver toxicity.

While 3D aggregated liver cells have been reported, the disclosed compositions have very high functional activity such as albumin production (up to a 10-fold increase compared with conventional highest standard models using iPSC-derived hepatocytes), and allow for improved oxygen and/or nutrition supply due to the internal luminal structure, which allows for much longer culture (at least over 60 days) and a long-term testing platform useful for drug testing. The disclosed compositions may also be useful for production of plasma products like albumin, coagulation factor products for treatment of hypoalbuminemia, and for therapeutic transplantation, in which the human iPSC-derived miniature livers can be transplanted to treat disorders in vivo. Lastly, the disclosed compositions may be used for personalized medicine (therapy personalization).

In one aspect, a method of inducing formation of a liver organoid from iPSC cells is disclosed. The method may comprise the steps of
a) contacting definitive endoderm (DE) derived from iPSC cells with a FGF pathway activator and a GSK3 inhibitor, for a period of time sufficient to form posterior foregut spheroids, preferably for a period of time of from about 1 day to about 3 days and b) incubating the resulting posterior foregut spheroids of step a in the presence of retinoic acid (RA) for a period of time sufficient to form a liver organoid, preferably for a period of time of from about 1 to about 5 days, preferably about 4 days.

Fibroblast growth factors (FGFs) are a family of growth factors involved in angiogenesis, wound healing, and embryonic development. The FGFs are heparin-binding proteins and interactions with cell-surface associated heparan sulfate proteoglycans have been shown to be essential for FGF signal transduction. Suitable FGF pathway activators will be readily understood by one of ordinary skill in the art. Exemplary FGF pathway activators include, but are not limited to: one or more molecules selected from the group consisting of FGF1, FGF2, FGF3, FGF4, FGF10, FGF11, FGF12, FGF13, FGF14, FGF15, FGF16, FGF17, FGF18, FGF19, FGF20, FGF21, FGF22, and FGF23. In some embodiments, siRNA and/or shRNA targeting cellular constituents associated with the FGF signaling pathway may be used to activate these pathways.

In some embodiments, DE culture is treated with the one or more molecules of the FGF signaling pathway described herein at a concentration of 10 ng/ml or higher; 20 ng/ml or higher; 50 ng/ml or higher; 75 ng/ml or higher; 100 ng/ml or higher; 120 ng/ml or higher; 150 ng/ml or higher; 200 ng/ml or higher; 500 ng/ml or higher; 1,000 ng/ml or higher; 1,200 ng/ml or higher; 1,500 ng/ml or higher; 2,000 ng/ml or higher; 5,000 ng/ml or higher; 7,000 ng/ml or higher; 10,000 ng/ml or higher; or 15,000 ng/ml or higher. In some embodiments, concentration of signaling molecule is maintained at a constant throughout the treatment. In other embodiments, concentration of the molecules of a signaling pathway is varied during the course of the treatment. In some embodiments, a signaling molecule in accordance with the present invention is suspended in media comprising DMEM and fetal bovine serine (FBS). The FBS can be at a concentration of 2% and more; 5% and more; 10% or more; 15% or more; 20% or more; 30% or more; or 50% or more. One of skill in the art would understand that the regiment described herein is applicable to any known molecules of the signaling pathways described herein, alone or in combination, including but not limited to any molecules in the FGF signaling pathway.

Suitable GSK3 inhibitors will be readily understood by one of ordinary skill in the art. Exemplary GSK3 inhibitors include, but are not limited to: Chiron/ CHIR99021, for example, which inhibits GSK3β. One of ordinary skill in the art will recognize GSK3 inhibitors suitable for carrying out the disclosed methods. The GSK3 inhibitor may be administered in an amount of from about 1 uM to about 100 uM, or from about 2 uM to about 50 uM, or from about 3 uM to about 25 uM. One of ordinary skill in the art will readily appreciate the appropriate amount and duration.

In one aspect, the stem cells may be mammalian, or human, iPSCs.

In one aspect, the foregut spheroids may be embedded in a basement membrane matrix, such as, for example, the commercially available basement membrane matrix sold under the tradename Matrigel.

In one aspect, the liver organoids may be characterized in that the liver organoids may express alpha-fetoprotein (AFP), albumin (ALB), retinol binding protein (RBP4), cytokeratin 19 (CK19), hepatocyte nuclear factor 6 (HNF6), and cytochrome P450 3A4 (CYP3A4), HNF4a, E-cadherin, DAPI, and Epcam. Such expression may occur, for example, at day 40 to day 50. The expression level may be similar to that observed in human liver cells, for example, that of an adult liver cell.

In one aspect, the liver organoid may be characterized in that the liver organoid has bile transport activity.

In one aspect, the liver organoid may be derived from a stem cell and may comprise a luminal structure further containing internalized microvilli and mesenchymal cells. The luminal structure may be surrounded by polarized hepatocytes and a basement membrane. The liver organoid may comprise functional stellate cells and functional Kupffer cells.

The liver organoid may, in certain aspects, be characterized by having one or more of the following: bile production capacity, bile transport activity, Complement factor H expression of at least 50 ng/mL/1xe⁶ cells/24hr, Complement factor B of at least 40 ng/ml/1xe⁶ cells/24hr, C3 expression of at least 1000 ng/mL/1xe⁶ cells/24hr; C4 expression of at least 1000 ng/mL/1xe⁶ cells/24hr, fibrinogen production of at least 1,000 ng/mL/1xe⁶ cells/24hr and albumin production of at least 1,000 ng/mL/1xe⁶ cells/24hr. In one aspect, the liver organoid may be characterized by having total hepatic protein expression of at least 10,000 ng/mL 1xe⁶ cells/24 hours. The liver organoid may be characterized in that it may express one or more genes selected from PROX1, RBP4, CYP2C9, CYP3A4, ABCC11, CFH, C3, C5, ALB, FBG, MRP2, ALCAM, CD68, CD34, CD31. In one aspect, the liver organoid may comprise cells comprising a drug metabolism cytochrome variant, such as, for example, a CY2C9*2 variant. The liver organoid may comprise a vasculature, such as that described in US 20160177270.

In one aspect, the liver organoid may be characterized in that the liver organoid does not comprise inflammatory cells, for example T-cells or other inflammatory secreted proteins.

In one aspect, a method of screening for a serious adverse event (SAE) is disclosed. The SAE may be liver failure and/or drug induced liver injury (DILI). The method may include the step of contacting a drug of interest, of which toxicity is of interest, with a liver organoid as described herein. In one aspect, the method may comprise the step of measuring intake and/or efflux of fluorescein diacetate (FD), wherein impaired efflux indicates that said drug is likely to induce a serious adverse event. The toxicity of a drug of interest may be determined by measurement of a parameter selected from mitochondria membrane potential, measurement of ROS, swelling of liver mitochondria, and combinations thereof, wherein injury to said mitochondria indicates that said drug is likely to induce a serious adverse event. In one aspect, the method comprises the step of assaying organoid viability, wherein impaired or decreased organoid viability indicates that said a drug of interest is likely to induce a serious adverse event.

In one aspect, a method of treating an individual having liver damage is disclosed, wherein the method may comprise the step implanting a liver organoid as described herein into an individual in need thereof. The liver damage may include, for example, metabolic liver disease, end stage liver disease, or a combination thereof.

In one aspect, methods for identifying a preferred therapeutic agent for an individual are disclosed. In this aspect, the method may include the step of contacting a liver organoid derived from an iPSC of interest with a candidate compound, such as wherein the iPSC of interest comprises one or more mutations found in said individual, or such as wherein said iPSC of interest is derived from the same ethic background of said individual, or further, wherein said iPSC of interest is derived from said individual.

### Examples

In the present study, Applicant tested bile transport activity using Fluorescein Diacetate, which was excreted by MRP2 across the canalicular membrane into the bile canalicular networks (Tian et al., 2004). It has previously been reported that Troglitazone and Cyclosporin inhibit the MRP2 (Chang et al., 2013; Lechner et al., 2010). In addition, the efflux transporter MRP2 mediates export of Bosentan (Fahrmayr et al., 2013). Although the inhibition of MRP2 by Nefazodone was not reported, mitochondria stress by Nefazodone may be related to a decrease of the bile transport activity, efflux of Fluorescein Diacetate, because MRP2 is an ATP-dependent bile salt transporter for canalicular excretion of bile acids in hepatocytes.

Preclinical detection of risk compounds for drug induced liver injury (DILI) remains a significant challenge in drug development, highlighting a need for a predictive human system. Here, Applicant developed a human liver organoid (HLO) model for analyzing clinical DILI pathology at organoid resolution. Differentiated HLO from human iPSC contain polarized hepatocytes with an internal lumen lined by bile canaliculi-like architecture, establishing the unidirectional bile acid transport pathway. Applicant has leveraged the organoid's structural features by modeling DILI using live organoid imaging, called LoT (Liver organoid-based Toxicity screen). LoT is functionally validated with 10 marketed drugs and 5 different donors based on cholestatic and/or mitochondrial toxicity. Bosentan-induced cholestasis is specific to CYP2C9 poor metabolizer donor-derived HLO. Interestingly, steatotic organoids were vulnerable to Rosiglitazone toxicity as suggested in clinics, followed by chemical rescue from massive organoid death. Thus, LoT is a high-fidelity organoid model that can be used to analyze drug safety, and is further a cost-effective platform, facilitates compound optimization, provides mechanistic studies, and produces personalized medicine as well as anti-DILI therapy screening applications.

Billions of dollars are lost annually from drug development in the pharmaceutical industry due to the failures of drug candidates identified in initial screens, and nearly (a third or one-third) of drugs are withdrawn from the market (Takebe and Taniguchi, 2014). Despite the promising efficacy, a failure of drug candidates results in a tremendous loss of a patient's treatment opportunity. Preclinical studies generally consist of an *in vitro* evaluation as a primary efficacy screen to identify a "hit" compound, followed by safety studies *in vitro* and *in vivo* to assess the mechanisms of metabolism and toxicology. This inefficiency can be explained by the substantial lack of physiologically relevant preclinical models in evaluating drug induced liver injury (DILI) in humans and thus, an urgent need to develop an in vitro humanistic screen model for the evaluation of the vast amounts of continuously growing compound libraries.

Primary hepatocytes are a highly polarized metabolic cell type, and form a bile canaliculi structure with microvilli-lined channels, separating peripheral circulation from the bile acid secretion pathway. The most upstream aspects of DILI include drug (or their reactive metabolites) detoxification by hepatocytes and excretion into bile canaliculi through transporters such as multi-drug resistance-associated protein (MRP) transporters. This suggests the need to reconstruct these uniquely organized structures as a crucial property of hepatocytes *in vivo* for predicting DILI pathology. However, there are considerable differences in drug toxicity profiles between the current simplified culture model with the use of isolated primary human hepatocytes or hepatic cell lines, and in vivo physiology, resulting in failed translation of drugs or drug discontinuation, as in the case of Troglitazone, Nefazodone and Tolcapone (https://livertox.nlm.nih.gov/index.html). The determination of toxicological properties thus mainly relies on animals as an essential step for drug development, however, due to the pronounced differences in physiology between humans and animals, there is a significant lack of fidelity to human outcomes (Leslie et al., 2007; Yang et al., 2014). In addition, the onset of idiosyncratic DILI (IDILI), which is very rare but nonetheless responsible for about 10-15% of acute liver failures in the USA (Reuben et al., 2010), is almost impossible to predict (Kullak-Ublick et al., 2017). Collectively, effective human cell models are eagerly anticipated to screen for compounds that test the detoxification and excretion of proposed drugs.

Despite the progressive advance of human hepatocyte differentiation methods from pluripotent stem cell (PSC), clinical trials in a dish using human stem cells remains 'hype'. To a certain degree, this can be explained by challenges in previous cell-based approaches including: (1) overcoming lot-differences, (2) minimization of experimental batch-differences, (3) enhancement of assay throughput and (4) improvement in relevance to clinical trial data. Applicant addresses these issues by developing a relatively simple and robust organoid based testing platform using stably expandable human stem cells, i.e. iPSC. Applicant first directed the human PSCs into posterior foregut organoids, followed by progressive hepatocyte differentiation through a polarization culture with defined factors and matrix. Generated human liver organoids possessed intraluminal structure surrounded by polarized hepatocytes, and have shown to be capable of performing critical human hepatocyte functions including protein and bile acid production and transport functions. Interestingly, Applicant found that live image based dynamic detection of fluorescent diacetate uptake and excretion precisely models cholestasis induced by arrays of DILI drugs characterized as inhibitors for bile excretion with a high level of reproducibility. Separately, mitochondrial membrane potential assessment enabled an independent risk assessment for each of the compound, reflecting a conventional classification of DILI drugs established by clinical trials. Furthermore, Applicant extended the approach to model conditions induced by lipotoxic stress and confirmed enhanced DILI potential through reactive oxygen species (ROS) production. Organoid-based viability assessment confirmed the reversal of DILI by N-acetylcysteine, highlighting the potential of our approach for anti-DILI drug screening. Taken together, this robust assay, named Liver Organoid based Toxicity screen (LoT), is believed to be the first functional readout developed in human liver organoids, and will facilitate diagnosis, functional studies, drug development and personalized medicine.

### RESULTS

Generation and characterization of polarized liver organoids from multiple human iPSC

Applicant first established a new liver organoid differentiation method by using human iPSC-derived foregut spheroids (Spence et al., 2011) (Figure 1A). As a first step, Applicant used BMP and Activin A to promote differentiation into definitive endoderm as previously described (D'Amour et al., 2005). In addition, FGF4, and a GSK3 inhibitor (CHIR99021) were used to induce foregut spheroids and budded spheroids were observed. Organoids were embedded in Matrigel after delamination with mesenchymal cells plated on the dish by gentle pipetting. It has been reported that retinoic acid (RA) enhances cell polarity as indicated by increased size and complexity of the bile canaliculi and the pericanalicular sheaths (Falasca et al., 1998). To generate polarized organoids suited for bile transport modeling, organoids were treated with RA. To optimize the organoid generation method, Applicant first varied the duration of RA treatment. The albumin secretion levels of organoids were 1160, 1054, 3092, 4709 and 3865 ng/mL at D25, for 1, 2, 3, 4 and 5 days of RA treatment, respectively, and the 4day-RA treatment protocol tended to reach the highest level (FIG 8). Thus, duration of RA was set for 4 days based on the level of albumin secretion. Morphologically, around 10 days after RA treatment, over 300 organoids covered with epithelial cells were successfully generated, and the ratio of organoids with lumenized structure was 71 % (216/305) (FIG 1, panel B and FIG 9). Immunohistochemistry analysis revealed that albumin was positive in epithelial cells of organoids, and interestingly, Type IV collagen was localized to the outer surface and ZO-1 (zonula occludens) stained the intraluminal lining, suggesting that these organoids have polarized characteristics (FIG 1, panel C).

Quantitative polymerase chain reaction (qPCR) analysis revealed that cells in organoids had a significant increase in expression of hepatic marker genes such as alpha-fetoprotein (AFP), albumin (ALB), retinol binding protein 4 (RBP4), Cytokeratin 19 (CK19), hepatocyte nuclear factor 6 (HNF6) which controls cholangiocyte differentiation, and Cytochrome P450 3A4 (CYP3A4) during differentiation (FIG 1, panel D). However, the expression level of the most hepatic genes extracted from bulk organoid-derived RNA was lower in organoids than in primary hepatocytes. Without intending to be limited by theory, it is believed that these distinct mRNA profiles are partly due to the presence of stromal lineages as approximately 30% of cells are non-parenchymal cells which were identified by stromal cell markers (Unpublished observation), making the organoids more similar to *in vivo* liver tissue than primary hepatocytes. Applicant further profiled the organoids by a comprehensive gene expression analysis using RNA-sequence (RNA-seq). Principal component analysis demonstrated that gene expression in organoids was not similar to iPSC-derived cholangiocytes and normal human cholangiocyte (FIG 1, panel E). Additionally, hepatocyte specific proteins such as ALB, fibrinogen (Fbg) and complement factors were confirmed in culture supernatant by ELISA (FIG 1, panels F-G). To quantitate the hepatic functionality of the organoids, Applicant investigated the albumin secretion level normalized by cell number (FIG 10). The albumin secretion level was 2133 ng/day/10⁶ cells (FIG 1, panel F) and higher than other experiments in 2D and 3D differentiation of hPSCs into HLC (150 -1000 ng/day/10⁶ cell) relative to published iPSC-derived hepatocytes (Miki et al., 2011; Song et al., 2015; Song et al., 2009; Vosough et al., 2013), while primary hepatocytes produce 30-40 µg/day/10⁶ cell in 3D scaffolds (Davidson et al., 2016; Dvir-Ginzberg et al., 2003). These results indicated that liver organoids contained hepatocytes with reasonable albumin secretion activity compared to stem cell-derived hepatocytes in the published literature. Importantly, this organoid generation method is reproducible and therefore, applicable to other PSC lines, as intra-luminal organoids were generated from both 317D6 and 1383D6 iPS cell lines with albumin secretion capacity (FIG 11). Overall, Applicant established a protocol for generating a large number of polarized liver organoids with hepatocyte characteristics.

### Micro-anatomical characterization of bile acid producing human iPSC-liver organoids

Next, to test if liver organoids have bile transport activity, Applicant first characterized organoids by staining key proteins involved in bile synthesis and excretion function. Immunofluorescence staining of BSEP and MRP2 demonstrated that these proteins preferentially localized in the intraluminal region (FIG 2, panel A). The bile canaliculus is the smallest intrahepatic secretory channel and the canalicular lumen consists of a space formed by a modified apical region of the opposing plasma membranes of contiguous hepatocytes (Cutrin et al., 1996; Tsukada et al., 1995). In addition, it is delimited by tight junction complexes and the microvilli are located on the inside of the canalicular lumen (Tsukada et al., 1995). ZO-1 staining is known to stain canalicular region in liver, and FIG 1, panel C suggested that tight junctions were located inside of our liver organoids. Transmission electron microscopy revealed organoids contained microvilli directed towards the lumen (FIG 2, panel B). Consistent with these anatomical features, qRT-PCR analysis revealed that organoids had gene expression of ABCB 11 and Na+-taurocholate co-transporting polypeptide (NTCP), yet the levels were lower in organoids than primary hepatocytes (FIG 2, panel C). Therefore, the organoid contained polarized human hepatocytes separated from internal lumen by tight junctions, which reflects a unique micro-anatomical architecture resembling *in vivo* hepatic canaliculi.

Next, in order to determine bile acid (BA) production capacity, Applicant conducted ELISAs on intra-luminal fluid collected from organoid culture. The level of total BA pool of intra-luminal fluid was 26.7 µg/day/10⁶ cells (approximately 125 µmol/L in an organoid with a 200 µm diameter) (FIG 2, panel D) and, surprisingly, the BA concentration was comparable to that in primary hepatocytes derived from sandwich culture (approximately 40 µg/day/10⁶ cells, 10 µmol/L in culture supernatant) in previous reports (Ni et al., 2016). Thus, organoids do not merely have the canaliculi-like morphology but possess bile acid production and secretion activity, suggesting that the bile acids transport pathway is correctly constructed.

### Dynamic visualization of bile acid intake and excretion in human liver organoids

Bile acid excretion is the major determinant of bile flow, therefore, defects in this system may result in impaired bile secretion (cholestasis) associated with various liver disease pathologies (Nishida et al., 1991). Efflux transport proteins located in the apical (canaliculi) membranes of hepatocytes play an important role in the hepatic elimination of many endogenous and exogenous compounds, including drugs and metabolites (Kock and Brouwer, 2012). BSEP and MRP2 mediate canaliculi bile salt transport in humans. After demonstrating the positive expression of key proteins for bile transport, the applicant next wondered if the organoids can actively transport bile acid into its lumen. First, to check the intake of bile acids into the organoid, applicant challenged the organoids with cholylglycylamido-fluorescein (CGamF), which is a bile salt analog (Mork et al., 2012). After treatment of CGamF from outside, the accumulation of CGamF into the intra-lumen of organoids was successfully confirmed (FIG 2, panel E). Similarly, the fluorescent bile acid cholyl-lysyl-fluorescein (CLF) was found to be reproducibly excreted and accumulated into organoids from multiple human iPSC lines (FIG 2, panel F). To determine the specificity of this assay, Applicant has developed an iPSC line carrying a BSEP defunctionalized allele using the CRISPR-Cas9 based gene editing approach. BSEP is responsible for bile transport, and consistent with this, BSEP-KO iPSC-organoids failed to accumulate fluorescent bile acid compared with parental control organoids. Taken together, these data suggest that organoids have the ability to uptake bile acid from the outside and efflux them inside the organoids.

### Bosentan induced cholestasis specific to CYP2C9*2 iPSC-liver organoids

To test the clinical relevance of organoid based cholestatic phenotyping method, Applicant employed pharmacogenomics insights into our system to address the fidelity question. Specifically, multiple iPSC lines have been collected, which carry a well-known susceptibility gene variant (i.e. CYP2C9*2 for Bosentan, described in, for example, Clin Pharmacol Ther. 2013 Dec;94(6):678-86. doi: 10.1038/clpt.2013.143. Epub 2013 Jul 17. Association of CYP2C9*2 with bosentan-induced liver injury.) (FIG 3, panel A), and compared their cholestatic potential in the presence of Bosentan (FIG3, panel B). Interestingly, CLF excretion into organoids was severely impaired in CY2C9*2 carrier organoids but not in non-carrier organoids. This matches the clinical tendency to cholestasis induced by Bosentan, shown in three-different iPSC derived organoids in the absence of CYP2C9*2 as shown in FIG 3, panel C. In contrast, irinotecan based cholestasis was not specific to CYP2C9*2 iPSC lines. These results indicated the organoid based cholestasis assay predicts some aspects of human variation.

### High-throughput drug induced cholestasis evaluation in organoids

Considering the significant role of cholestasis in DILI induced by drugs, the applicant next wondered if this organoid model reflects the pathology of DILI in the presence of particular compounds. Before testing a number of compounds, the applicant first sought to develop a high-throughput fluorescence based assay because both CLF and CGamF are not applicable for high-speed imaging due to several issues: 1. background is strong, necessitating a manual washing process; 2. signal intensity is weak, requiring a careful acquisition setting. Alternatively, the use of fluorescein diacetate (FD), a reportedly useful marker of efflux transport in hepatocytes (Barth and Schwarz, 1982; Bravo et al., 1998) is proposed. The polar fluorescent metabolite fluorescein, is trapped in the cells until it is actively transported from the cells into the canaliculi space (Malinen et al., 2014). To determine if FD can be used for live assessment of transport capacity without exchanging the medium and adjusting exposure, chronological hepatobiliary transport activity was further investigated with time-lapse imaging. Organoids were incubated with fluorescein diacetate for 45 minutes and intraluminal accumulation was observed inside of organoids at 20 minutes after treatment (FIG4, panels A, B). The opposite directionality of this transport flow was determined by the micro-injection of FD into organoids. After micro-injection of diacetate into the lumen, fluorescein remained inside, and was never observed outside the organoids (FIG 4, panel C). In summary, this FD based evaluation model has high-throughput potential to assess unidirectional efflux bile transport in liver organoids by a simple fluorescent live imaging analysis.

Next, the applicant validated the fidelity of a FD-based assay by evaluating the viable dosing of 10 FDA approved drugs and measured any secondary disturbance by cell damage. Applicant successfully found an optimal dose for nine compounds with acceptable viability. In contrast, amiodarone (AMIO) was significantly toxic to organoids within the tested range, therefore AMIO was excluded in further potential DILI assessment studies (FIG 12). Applicant investigated cholestasis potential in organoids using FD with nine training compounds (TCs) which were classified as one of three types based on DILI mechanism; DILI compounds without cholestasis (Class A), DILI compounds with cholestasis (Class B) and compounds not reported as DILI compounds (Class C) (FIG 4, panel D) (Oorts et al., 2016). To quantify the inhibitory potential for FD excretion, Applicant developed a simple but robust quantification method by determining the fluorescent intensity ratio between outside and inside the organoid by image J (FIG 4, panel B). As a validation study, Applicant first confirmed the ability to assess the inhibition ratio using Cyclosporin A (CSA). At five minutes after treatment of FD, a significant decrease (0.4 compared to control) was observed in the group treated with CSA for 24h, compared to control (DMSO) (FIG 4, panel B). Applicant then screened nine TCs at multiple concentrations to assess the fidelity of this approach. Interestingly, in this screening system, at 24hr after treatment of TCs, the efflux of FD was significantly decreased (p < 0.01 or 0.05) in Class B compounds) Bosentan, CSA, Troglitazone and Nefazodone), similar to clinical observations, while this inhibitory effect was not observed in Class A and Class C compounds (FIG 4, panel D upper images and FIG 4, panel E). These results suggested that the liver organoid model is useful for classifying the bile transport inhibition potency for candidate compounds in drug development with a high relevance to human phenotypes.

### Evaluating mitochondrial overload in organoids

Further, Applicant investigated the mitochondria health assessment because mitochondrial toxicity plays a central role in DILI in multiple mechanisms associated with the onset of DILI (Pessayre et al., 2012). In this study, to investigate mitochondrial health in organoids, an index of mitochondrial membrane potential (MMP) was used to monitor MMP of intact cells as a direct readout of mitochondrial health (Li et al., 2014). After treatment of TCs for 24h, dose-dependent increases of MMP were observed with treatment of Tolcapone (2-8-fold change, p < 0.01), Diclofenac (7-13 Fold change, p < 0.05 or 0.01), CSA (3-7-fold change, p < 0.01) and Nefazodone (4-42 fold change, p < 0.01) (FIG 5, panel A lower images and graph). In addition, Troglitazone also increased MMP in organoids (3-5-fold change, p < 0.05), although dose-dependence was not observed. On the other hand, after treatment of Bosentan, Entacapone and Pioglitazone, increases of MMP were not clearly observed even in multiple doses. These results demonstrated that this live image based assay, named Liver organoid-based Toxicity screen (LoT), discriminated between compounds with and without mitochondrial toxicity.

### Revisiting mechanistic classification of DILI compounds by LoT system

Severe manifestations of human DILI are multifactorial, highly associated with combinations of drug potency specifically related to known mechanisms of DILI such as mitochondrial and BSEP inhibition (Aleo et al., 2014). However, current *in vitro* functional models are difficult to assess such multi-factorial contributions. Given the advantage of multiplexed and live functional readouts in LoT system, Applicant attempted to analyze the relationship among survival, cholestasis and mitochondrial stress. Of note, drugs with dual action at 24 hours (cholestasis and mitochondrial stress) such as CSA, TRO and NEFA significantly lowered cell viability at 72 hours relative to TOL, DICLO and BOS. These data are comparable to clinical data that shows that dual toxicities were highly associated with the severity of DILI consistent previous reports (Aleo et al., 2014) (FIG 5, panels B, C). Additionally, Applicant also noted that Entacapone treatment at 130 µM decreased organoid viability (From 85% at 24h to 64% at 72h). Entacapone requires extensive binding to plasma proteins, mainly to albumin, to induce DILI (Fisher et al., 2002). However, based on available methods, it remains elusive how Entacapone is toxic to the liver (Oorts et al., 2016). Taken together, the LoT system is an advantageous human model system for the major mechanistic classification of DILI, and a useful testing platform for further delineating unknown complex mechanisms.

### Assessing vulnerability for DILI in human liver organoids

DILI incidence is known to be often confounded by a number of host factors. Indeed, there is growing evidence that the risk of hepatotoxicity from some drugs such as acetaminophen is greatly increased due to obesity and NAFLD, both in rodents and humans (APAP) (Fromenty, 2013; Michaut et al., 2016). Therefore, it is important to predict DILI potential in such a "vulnerable" condition with a patient even in the subclinical phase. In the present study, Applicant established a lipotoxic organoid model by co-exposure to an unsaturated fatty acid, oleic acid (FIG 6, panel A). At 3 days after oleic acid treatment to organoids, lipid accumulation in organoids was intense (FIG 6, panel B). The oxidation of fatty acids is an important source of reactive oxygen species (ROS), which leads to depletion of ATP and nicotinamide dinucleotide, and induces DNA damage in fatty livers (Browning and Horton, 2004). Consistent with this, ROS production was observed in lipid treated organoids (FIG 6, panel C and FIG 13, panel A). Additionally, fatty acids induced massive swelling of liver mitochondria (FIG 6, panel D and FIG 13, panel B) similar to published phenotypes (Zborowski and Wojtczak, 1963). As hepatic mitochondrial dysfunction precedes the development of NAFLD in a rat model (Rector et al., 2010), these results indicate that the lipotoxic organoids model to some degree *in vivo* fatty liver model.

Recognizing this lipotoxic organoid model as a vulnerable condition with enhanced ROS production, Troglitazone (0-50 µM) was treated for 24h and cell viability in organoids was assessed. By treatment of Troglitazone alone at 50 µM, cell viability was 85 % at 24 hours while it was decreased to 67 % at 72h. However, after treatment of Troglitazone on lipotoxic condition, massive fragmentation of organoids was observed due to an organoid death. Subsequent cell viability analysis confirmed this result (-40 % compared to control, p < 0.05) (FIG 6, panel E and 6, panel F).

Next, Applicant investigated whether organoids can be recovered from a DILI-like condition by a potential therapeutic compound. The applicant used N-acetylcysteine (NAC), an antioxidant, to inhibit ROS production because intravenous NAC improves survival in patients with non-acetaminophen-related acute liver failure (Lee et al., 2009) and diminished the Troglitazone-induced cytotoxicity (Rachek et al., 2009). As expected, cell viability was significantly improved by NAC, suggesting that NAC rescued cell death in organoids even in vulnerable conditions (FIG 6, panel E and 6, panel F). In most DILI cases, the only intervention is the removal of the causative drug if identified (Polson and Lee, 2005) (Bohan et al., 2001; Navarro and Senior, 2006). This LoT system may be a useful tool for identifying causal drugs linked to multi-drug regimens as well as drug discovery for treating DILI.

Serious adverse events (SAE) including liver failure are a major cause of drug attrition during clinical development or withdrawal of marketed pharmaceuticals. In particular, DILI is a critical challenge in drug development, in which drug induced cholestasis induced by inhibitions of transporter activity is one major cause. Sandwich culture using human primary hepatocytes is the current best choice in pharmaceuticals. Although recent reports showed the promise of hepatocyte based cholestasis model using transdifferentiated cells from human fibroblasts (Ni et al., 2016), these assay platforms still have reproducibility challenges as well as throughput problems because of variable and limited human hepatocyte sources and the need for complex quantitation algorithms. In addition, HepaRG cells, a human hepatoma cell line, is also useful to evaluate cholestatic features, but their low BSEP (Bile Salt Export Pump, or ABCB11, important transporter for bile acid excretion, as well as major target for cholestatic agents) activity and time-consuming differentiation procedure, limit their use (Le Vee et al., 2013). More importantly, a lack of essential anatomical structures limits their practical use for the pharmaceutical industry. Alternatively, the described methods allow for a simple, robust and high-throughput system to measure bile transport activity by live fluorescent imaging in the presence of testing compounds. The major advantages of the LoT assay include: 1. the cost effectiveness ($12.35 per 50 organoids; $94.85 per 384 well), 2. the assay throughput (measurable at single organoid) and 3. the multiplexed readouts for analyzing interplay between other factors such as mitochondrial stress. Especially, as mentioned above, retrospective studies revealed that multiple cell stress potentials were associated with the incidence of DILI (Aleo et al., 2014) and the LoT assay showed the comparable results with that study, since cell viability was decreased dependent on dual readouts; mitochondrial and cholestatic stress. Oxidative stress plays an important role in cell death and has been linked to the development of cholestatic liver injury (Serviddio et al., 2004). Hydrophobic bile acids accumulate intracellularly during cholestasis and interfere with normal mitochondrial electron transport, inhibiting the activity of respiratory complexes I and III and consequently reducing adenosine triphosphate synthesis (Krahenbuhl et al., 1994), resulting in mitochondrial dysfunction-induced apoptosis (Bernardi, 1996). In line with these findings, Applicant's correlational analysis of these dual readouts indicated cholestatic stress was a more dominating factor for liver injury compared with mitochondria stress as was seen in FIG 5. Thus, the LoT system may be used as a model system for investigating DILI mechanisms.

In addition, given the recent establishment of population of iPSC panels, potential assessment for different susceptibility in individuals is also promising (Inoue et al., 2014). Predicting SAE in conventional *in vitro* assay system does not generally focus on individual differences, however, SAE often occur in a small SAE-prone patient subgroup (Stevens and Baker, 2009). Applying the LoT system to diverse population iPSC panels will provide previously inaccessible about different susceptibilities to SAE. In light of the extremely rare nature of DILI, the use of patients' cells with specific genomic or ethnic factors will aid in elucidating the currently unknown idiosyncratic mechanism of DILI. Thus, LoT might serve as a game changing strategy for pharmaceutical industries by providing essential insight for minimizing DILI potential (FIG 7).

One limitation in this organoid model is the lack of immunological reactions. The immunological effect resulting from a hypersensitivity reaction is one possible mechanism for idiosyncratic DILI. There are limited *in vitro* models to assess the hypersensitivity by drugs, although the sensitivity by Troglitazone-induced cytotoxicity was increased using in vitro co-culture model using hepatic cell line, Huh7 and THP-1 cells (Edling et al., 2009). Thus, advancing the LoT platform by focusing on immune lineages will be of interest to assess hepatocellular inflammation. Nevertheless, the LoT testing platform seems superior in generating reproducible and large data sets from an individual organoid as the inhibition of bile efflux function by multiple FDA approved drugs is reproducibly observed in this assay. Considering cholestasis is induced by a broad spectrum of liver diseases including drug-induced, lipotoxic, infectious, and congenital conditions (Chatterjee et al., 2014), the organoid based LoT assay is useful for analyzing intra-hepatic cholestasis in a variety of contexts with the potential for mechanistic studies as well as drug screening applications beyond DILI.

### Studying vulnerable human liver condition with LoT assay

Host factors such as obesity are known to significantly influence the onset of DILI (Heidari et al., 2014), yet they are often understudied in the clinical setting due to its complex nature. The presence of obesity or fatty liver might make patients vulnerable to hepatic injury induced by xenobiotics and non-toxic chemicals (*e.g.,* drugs) and these might become hepatotoxic in lower doses in the presence of risk factors (Fromenty, 2013). Despite this, the current clinical trial system is not designed for stratifying volunteers under vulnerable liver conditions with handful of biomarker (ALT, AST) levels. Since number of patients with steatosis are subclinical with no detectable biomarkers before dosing, it is critical to foresee the outcomes in this vulnerable condition before entering the clinical phase.

With an effort to advance the LoT system to assess the toxicity in these vulnerable conditions on early drug screening stage such as lead compound generation/optimization, Applicant applied lipotoxic stress to liver organoids and demonstrated the exaggerated synergistic effect of Troglitazone, an antidiabetic drug, on DILI. Indeed, the organoid system successfully reflects this feature by showing massive hepatocyte death, promoted by triglyceride accumulation into hepatocytes in an organoid. One of the mechanisms of DILI in obesity could explain reduced levels of glutathione (GSH) (Michaut et al., 2016). Drug-induced oxidative stress could have several origins, in particular, through GSH depletion and inhibition of the mitochondrial respiratory chain (Begriche et al., 2011; Pessayre et al., 2010). The vulnerable model might reflect the decrease in intracellular GSH levels and worsened Troglitazone induced oxidative stress through mitochondrial dysfunction, ameliorated by providing NAC. Considering the dramatic rise in non-alcoholic steatohepatitis (NASH) prevalence, it is noteworthy that there is still a minimal list of drugs for either aggravating pre-existing NAFLD or inducing more frequently an acute hepatitis. Additionally, an *in vitro* reductionist system provides a previously unforeseen window to study previously untested host factors as the isolated host factor can effectively deployed into organoids.

### LoT based precision medicine

The selection of optimal drug therapy using LoT will be of major interest in clinics from a personalized medicine perspective. For instance, the strategy considered in the choice of antipsychotic agent must take into account the hepatic tolerance according to the non-negligible incidence of liver disorders among psychiatric population; 16% of possible DILI agents are neuropsychiatric drugs (Dumortier et al., 2002). Given that NASH is often accompanied by psychological disorders such as depression, safer combinatorial selection of anti-depressive drugs (tricyclic agents or SSRI), mood stabilizing agents and neuroleptic drugs is needed (Dumortier et al., 2002). Also, due to an age-associated increase in chronic conditions, multiple medication use (*i.e.,* polypharmacy) is a common consequence of providing health care to older adults (Marcum and Gellad, 2012), making it extremely difficult to identify causative drugs when DILI is suspected. As patient derived iPSC-organoids provide an unlimited and reproducible source, LoT may serve as a panel to stratify the potential of DILI in patients and provide information to choose safer medication from a personalization perspective.

### LoT based new drug discovery to DILI

Equally important is the potential use for anti-DILI therapeutic compound screen using LoT system. A large number of drugs have deleterious effects on liver and DILI and it is a major clinical problem. Actually, acetaminophen accounts for approximately half of cases of DILI in the United States (Russo et al., 2004). In other regions of the world, for instance in developing countries, other drugs such as antituberculosis medications might be the leading cause of DILI (Bell and Chalasani, 2009). However, there are only several symptomatic therapies available. Here, as a proof-of-concept experiment, Applicant established an organoid survival experiment to assess the therapeutic effect of a compound to resist a toxic mechanism of DILI as evidenced by Troglitazone. While NAC is the major treatment choice for paracetamol overdose (Makin et al., 1995; Verma and Kaplowitz, 2009), recently, the research focus has shifted to investigating the use of NAC in non-paracetamol DILI (Chughlay et al., 2016). LoT system is useful to assess the efficacy of NAC to DILI by non-paracetamol drugs. Beyond, this higher throughput approach will serve as a powerful tool for screening larger scale compound libraries that restore DILI-like symptoms in vitro. Combined, methods described here can be used to identify and study cell-intrinsic and extrinsic factors associated with clinical DILI phenotypes, and would facilitate lead compound optimization, mechanistic study, and precision medicine, as well as anti-DILI therapy screening applications.

### METHODS

### Maintenance of PSCs

TkDA3 with CYP2C9*2 variant human iPSC clone used in this study was kindly provided by K. Eto and H. Nakauchi. Other suitable lines include human iPSC lines gifted from Kyoto University and those purchased from Coriell Biorepository. were maintained as described previously (Takahashi et al., 2007). Undifferentiated hiPSCs were maintained on feeder-free conditions in mTeSR1 medium (StemCell technologies, Vancouver, Canada). Other suitable media include E8 from Lonza, or StemFit from Aijinomoto Co. Plates are coated with Matrigel (Corning Inc., New York, NY, USA) of 1/30 dilution at 37°C in an incubator with 5% CO₂/95% air. hPSCs Maintenance. In place of Matrigel, Laminin511, Laminin411 from Mippi Co or Biolamina Co can be used.

### Production of Liver Organoids (HLO)

Differentiation of hiPSCs into definitive endoderm was induced using previously described methods with several modifications (Spence et al., 2011). In brief, colonies of hiPSCs were isolated in Accutase (Thermo Fisher Scientific Inc., Waltham, MA, USA) and 150000-300000 cells were plated on Matrigel or laminin coated tissue culture 24 well plate (VWR Scientific Products, West Chester, PA). When the cells become a high-density (over 90 % of the cells covering the well), medium was changed to RPMI 1640 medium (Life Technologies, Carlsbad, CA) containing 100 ng/mL Activin A (R&D Systems, Minneapolis, MN) and 50 ng/mL bone morphogenetic protein 4 (BMP4; R&D Systems) at Day 1, 100 ng/mL Activin A and 0.2 % fetal calf serum (FCS; Thermo Fisher Scientific Inc.) at Day 2 and 100 ng/mL Activin A and 2% FCS at Day 3. For Day 4-6, cells were cultured in Advanced DMEM/F12 (Thermo Fisher Scientific Inc.) with B27 (Life Technologies) and N2 (Gibco, Rockville, MD) containing 500 ng/ml fibroblast growth factor (FGF4; R&D Systems) and 3 µM CHIR99021 (Stemgent, Cambridge, MA, USA). Cultures for cell differentiation were maintained at 37°C in an atmosphere of 5% CO₂/95% air and the medium was replaced every day. Differentiated definitive endoderm showed budding on the plate at Day 7. If the spheroids were not enough to be embedded into Matrigel, Day4-6 media is added again and incubated with at 37°C overnight.

**Differentiation into liver organoids.** Three methods may be used to differentiate the DE into liver organoids: The "Matrigel Drop Method," the "Matrigel Sandwich Method," and the Matrigel-Free Method," each of which is described below.

**Matrigel Drop Method:** On Day 7-8, definitive endoderm organoids with plated cells were gently pipetted to delaminate from dishes. Isolated spheroids were centrifuged at 800 rpm for 3 minutes and, after removing supernatant, embedded in 100 % matrigel drop on the dishes. The plates were placed at 37°C in an atmosphere of 5% CO₂/95% air for 5-15min. After the Matrigel was solidified, Advanced DMEM/F12 was added with B27, N2 and Retinoic acid (RA; Sigma, St. Louis, MO) 2 µM for 1-5 days. The media was replaced every other day. After RA treatment, organoids embedded in Matrigel drop were cultured in Hepatocyte culture medium (HCM Lonza, Walkersville, MD) with 10 ng/mL hepatocyte growth factor (HGF; PeproTech, Rocky Hill, NJ), 0.1 µM Dexamethasone (Dex; Sigma) and 20 ng/mL Oncostatin M (OSM; R&D Systems). Cultures for cell differentiation were maintained at 37°C in an atmosphere of 5% CO₂/95% air and the medium was replaced every 3 days. Around Day 20-30, organoids embedded in Matrigel drop were isolated by scratching and gentle pipetting for any analyses.

**Matrigel Sandwich Method:** On Day 7-8, definitive endoderm organoids with plated cells were gently pipetted to delaminate from dishes. Isolated spheroids were centrifuged at 800 rpm for 3 minutes, and after removing supernatant, they were mixed with 100 % Matrigel. At the same time, hepatocyte culture medium with all supplements was mixed with the same volume of 100 % Matrigel. HCM and Matrigel mix was plated to the bottom of dish to make a thick coating on the plate (0.3-0.5 cm), and placed at 37°C in an atmosphere of 5% CO₂/95% air for 15-30 min. After the Matrigel was solidified, spheroids mixed with Matrigel was seeded on Matrigel thick coated plated. The plate was placed at 37°C in an atmosphere of 5% CO₂/95% air for 5 min. Advanced DMEM/F12 was added with B27, N2 and Retinoic acid (RA; Sigma, St. Louis, MO) 2 µM for 1-5 days. The media was replaced every other day. After RA treatment, organoids embedded in Matrigel drop were cultured in Hepatocyte culture medium (HCM Lonza, Walkersville, MD) with 10 ng/mL hepatocyte growth factor (HGF; PeproTech, Rocky Hill, NJ), 0.1 µM Dexamethasone (Dex; Sigma) and 20 ng/mL Oncostatin M (OSM; R&D Systems). Cultures for cell differentiation were maintained at 37°C in an atmosphere of 5% CO₂/95% air and the medium was replaced every 3 days. Around Day 20-30, organoids embedded in Matrigel drop were isolated by scratching and gentle pipetting for any analyses.

**Matrigel-Free Method:** On Day 7-8, definitive endoderm organoids with plated cells were continued planar culture in Advanced DMEM/F12 (Thermo Fisher Scientific Inc.) with B27 (Life Technologies) and N2 (Gibco, Rockville, MD) Retinoic acid (RA; Sigma, St. Louis, MO) 2 µM for 4 days. The media was replaced every other day. After the 4days planar culture, the organoids begin to bud, whereas 2D cells differentiate into hepatocytes. Both organoids and hepatocytes can be maintained for over 60 days under Hepatocyte culture medium (HCM Lonza, Walkersville, MD) with 10 ng/mL hepatocyte growth factor (HGF; PeproTech, Rocky Hill, NJ), 0.1 µM Dexamethasone (Dex; Sigma) and 20 ng/mL Oncostatin M (OSM; R&D Systems) for 10 days. For organoid assays, floating organoids can be collected in Ultra-Low attachment multiwell plates 6 well plate and used for subsequent assays whenever appropriate. Cultures for cell differentiation were maintained at 37°C in an atmosphere of 5% CO₂/95% air and the medium was replaced every 3 days.

### H&E staining and immunohistochemistry

Liver organoids were collected from Matrigel, fixed in 4% paraformaldehyde and embedded in paraffin. Sections were subjected to H&E and immunohistochemical staining. The following primary antibodies were used: anti-human albumin antibody (1:200 dilution abcam, Cambridge, UK), anti-type IV collagen antibody (1:200 dilution eBioscience, San Diego, CA, USA), anti-ZO-1 antibody (1:200 dilution BD Transduction Laboratories (San Jose, CA, USA) and anti-MRP2 antibody (1:200 dilution Novus Biologicals, Littleton, CO). Dye-conjugated secondary antibodies, Alexa Fluor 568-conjugated donkey anti-rabbit immunoglobulin (IgG; 1:1000; Invitrogen, A10042) was applied to the organoids at room temperature for 2 h. Nuclei were stained with 10 µg/mL Hoechst 33342 (Sigma) at room temperature for 10 min, after which organoids were washed again three times with washing buffer. The specimens were observed under a Fluorescent microscope or bright-field. For whole mount immunohistochemical staining, after liver organoids were fixed in 4% paraformaldehyde for 30 min and permeabilized with 2.5% Tween 20 (Sigma) at room temperature, organoids were incubated overnight at 4°C with the following primary antibodies diluted in PBS: polyclonal anti-BSEP antibody (1:200 Sigma). Fluorescent dye-conjugated secondary antibodies, Alexa Fluor 568-conjugated donkey anti-rabbit immunoglobulin (IgG; 1:500; Invitrogen, A10042) were applied to the organoids at room temperature for 2 h. After the reaction, the cells were washed three times with washing buffer (PBS containing 0.5% Triton-X 100 [Sigma] and 0.5% bovine serum albumin [BSA; Sigma]). Nuclei were stained with 10 µg/mL Hoechst 33342 (Sigma) at room temperature for 10 min, after which organoids were washed again three times with washing buffer. The specimens were observed under a confocal imaging performed on a Nikon A1Rsi inverted confocal microscope.

### RNA isolation, RT-qPCR

RNA was isolated using the RNeasy mini kit (Qiagen, Hilden, Germany). Reverse transcription was carried out using the SuperScriptIII First-Strand Sysnthesis Systen for RT-PCR (Invitrogen, CA, USA) according to manufacturer's protocol. qPCR was carried out using TaqMan gene expression master mix (Applied Biosystmes) on a QuantStudio 3 Real-Time PCR System (Thermo). All primers and probes information for each target gene were obtained from the Universal ProbeLibrary Assay Design Center (https://qpcr.probefinder.com/organism.jsp).

### Principal component analysis of RNA-seq data

RNA isolation, cDNA synthesis, sequencing on Illumina HiSeq 2500 are described previously (Asai et al., 2017). The RNA-Seq reads were aligned to the human genome (GRCh37/hg19) using TopHat (version 2.0.13). The alignment data from Tophat were fed to an assembler, Cufflinks (version 2.2.1), to assemble aligned RNA-Seq reads into transcripts. Annotated transcripts were obtained from the UCSC genome browser (http://genome.ucsc.edu) and the Ensembl database. Transcript abundances were measured in Fragments Per Kilobase of exon per Million fragments mapped (FPKM).

To compare the lineage of pHLO, Applicant combined in-house RNA-seq data (pFG and organoids) with preprocessed public data as follows: Transcript abundances of iPSC, DE, HS, HP, iDH and NHC were obtained from GSE86007 (Jalan-Sakrikar et al., 2016); ones of child liver tissue, adult liver tissue, adult right lobe tissue, fetal liver tissue and primary hepatocyte were obtained from ENCODE (ENCFF418BVF, ENCFF804QWF, ENCFF965IQH, ENCFF918SJO, ENCFF367FJJ, ENCFF029IUF, ENCFF280YNO, ENCFF347TXW, ENCFF724CQI, ENCFF624LQL, ENCFF962SOD, ENCFF170AEC) (Consortium, 2012; Sloan et al., 2016) and GSE85223(Asai et al., 2017). Genes were used if all dataset have identical gene symbol after possible data preprocessing. Applicant performed quantile-normalization of FPKM+1 and RPKM+1 data in log2 space followed by selected genes within the top 10000 of median expression levels. Principal component analysis was performed by using the scaled gene expression levels by using R package FactoMineR (version 1.35) (Sébastien Lê, 2008).

### Protein secretion zanalysis

For measuring albumin, fibrinogen and complement factors secreted level of organoids, 200 µL of culture supernatant of organoids on Ultra-Low attachment 96 well plates (Corning) were collected. The culture supernatants were collected and stored at - 80°C until use. The supernatant was assayed with Human Albumin ELISA Quantitation Set (Bethyl Laboratories, Inc., TX, USA) and fibrinogen (Thermo Fisher Scientific) according to the manufacturer's instructions. For analyzing complement factors, supernatants were measured with Luminex System (Luminex Corporation, Austin, TX) according to the manufacturer's instructions. To calculate albumin production per the number of cells, the linear regression equation of cell numbers by diameter of an organoid was used. For measuring total bile acid secreted level intra-luminal organoids, fluid inside organoids was absorbed using microinjection Nanoject II (Drummond Scientific, Broomall, PA, USA). Fluid absorbed was dilute in PBS and assayed with Total Bile Acid ELISA Kit (Antibodies-online, Inc., GA, USA). To calculate the volume of total bile acid, the number of cells in organoid was calculated using the linear regression equation in the same manner for albumin production and the molecular weight of cholic acid was used for calculation and comparing with volume in previous report.

### Transmission electron microscopy

For transmission electron microscopy, briefly, organoids were fixed in 3% glutaraldehyde for overnight at 4 °C, washed in 0.1 M sodium cacodylate buffer, and incubated for 1 h in 4% osmium tetroxide. They were subsequently washed then dehydrated in ethanol series, and finally embedded in propylene oxide/LX112. Tissue was then sectioned and stained with 2% uranyl acetate followed by lead citrate. Images were visualized on Hitachi transmission electron microscope.

### CGamF assay

Briefly, organoids were pre-incubated with a transport buffer (118 mM NaCl, 23.8 mM NaHCO3, 4.83 mM KCl, 0.96 mM KH2PO4, 1.20 mM MgSO4, 12.5 mM HEPES, 5 mM glucose, 1.53 mM CaCl2, adjusted to pH 7.4) for 30 min. Next, organoids were treated by 10 µM fluorescently labeled bile acid (CGamF; a kind gift from Dr Hofmann) for 1h, after then, organoids were washed three times with PBS. Images were captured on fluorescent microscopy BZ-X710 (Keyence, Osaka, Japan).

### Evaluation of bile transport inhibition

Fluorescein diacetate was used for evaluating bile transport activity in organoids. Around Day 25, the organoids were rinsed with PBS, and, fluorescein diacetate was treated to organoids in medium. In addition, to investigate the direction of transport, fluorescein diacetate was injected to organoids using by Nanoject III (Drummond Scientific). After treatment or injection of fluorescein diacetate, images were captured on fluorescent microscopy BZ-X710 (Keyence). Next, to check the feasibility of test system, 10 mg/mL fluorescein diacetate (Sigma) in HCM was added with 20 µM Cyclosporin A (CSA; Sigma) for 45 minutes and images were captured sequentially using fluorescent microscopy BZ-9000 (Keyence). For evaluation of bile transport inhibition, 10 mg/mL fluorescein diacetate in HCM was added after treatment of dimethyl sulfoxide (DMSO; Sigma), Streptomycin (STP; Sigma) as a negative control, Tolcapone (Tol; Sigma), Diclofenac (Diclo; Sigma), Bosentan (BOS; Sigma), CSA, Troglitazone (Tro; Sigma), Nefadozone (Nefa; Sigma), Entacapone (Enta; Sigma) and Pioglitazone (PIO, Sigma). After 5 minutes incubation, the organoids were rinsed three times with PBS and images were captured sequentially using fluorescent microscopy BZ-X710. Analysis was performed by calculating ratio between the intensities outside and inside organoids using Imagej 1.48k software (Wayne Rasband, NIHR, USA, http://imagej.nih.gov/ij). Changes in brightness or contrast during processing were applied equally across the entire image.

### Mitochondria toxicity potential evaluation

After being cultured in on Ultra-Low attachment multi-well plates 6 well plate in each culture condition, organoids were picked up and seeded in Microslide 8 Well Glass Bottom (Ibidi, WI, USA). For evaluation of mitochondria membrane potential (MMP), 250 nM Tetramethylrhodamine, Methyl Ester, Perchlorate (TMRM; Thermo Fisher Scientific) was added after treatment of dimethyl sulfoxide (DMSO; Sigma), Streptomycin (STP; Sigma) as a negative control, Tolcapone (Tol; Sigma), Diclofenac (Diclo; Sigma), Bosentan (BOS; Sigma), Cyclosporin A (CSA; Sigma), Troglitazone (Tro; Sigma), Nefadozone (Nefa; Sigma), Entacapone (Enta; Sigma) and Pioglitazone (PIO, Sigma) for 24h. After 30 minutes incubation, the organoids were rinsed three times with PBS and images were scanned on a Nikon A1 Inverted Confocal Microscope (Japan) using 60x water immersion objectives. Arias and intensity of TMRM were calculated as MMP by IMARIS8 (Bitplane AG, Switzerland). For assessment of cholestatic and mitochondrial stress, cell viability was measured by using the CellTiter-Glo^{®} luminescent cell viability assay (Promega, Mannheim, Germany) per organoid at 24h after treatment of drugs and confirmed not to decrease the viability in each dose for avoiding secondary change due to cell damage toward death.

### Analysis of relationship of cell viability in organoids with mitochondria and cholestatic stress

To demonstrate the relationship of cell viability with mitochondrial and cholestatic stress, first, indexes were setup using following formula; "Index = - (Sample value -Control value) x100" based on the value provided from mitochondrial and cholestatic stress assays. For analyzing cell damage associated with mitochondrial and cholestatic stress, at 72h after treatment of drugs, the ATP content per organoid was determined using the CellTiter-Glo^{®} luminescent cell viability assay (Promega). These data were shown as FIG 4, panel B using Infogr.am (http://infogr.am): a free, web-based tool.

### Evaluation of viability in organoids on vulnerable condition

The experiment was performed as shown in Figure 5A. After being excluded from Matrigel and washed, organoids were treated 800 µM oleic acid on Ultra-Low attachment multiwell plates 6 well plate (Corning) for 3days. Next, 50 µM of Troglitazone was treated with or without 50 µM NAC for 24h. Cell viability was performed by using the CellTiter-Glo^{®} luminescent cell viability assay (Promega). Images were captured sequentially using fluorescent microscopy BZ-9000.

### Lipid induced mitochondria stress evaluation

After being cultured in on Ultra-Low attachment multiwell plates 6 well plate in each culture condition, twenty organoids were picked up and seeded in Microslide 8 Well Glass Bottom (Ibidi, WI, USA) and subjected to live-cell staining. The following regents or kits were used: BODIPY^{®} 493/503 for lipids (Thermo Fisher Scientific), and SiR Actin Kit for cytoskeleton (USA Scientific, FL, USA), CellROX^{®} Green Reagent for ROS (Fisher Scientific), TMRM (Thermo Fisher Scientific) for mitochondria. Organoids were visualized and scanned on a Nikon Al Inverted Confocal Microscope (Japan) using 60x water immersion objectives. ROS production, mitochondria size and number were analyzed by IMARIS8.

### Statistics

Statistical significance was determined using unpaired Student's t-test or one-way ANOVA with Dunnett's multiple comparison post-hoc test. P < 0.05 was considered significant.

### References

Aleo, M.D., Luo, Y., Swiss, R., Bonin, P.D., Potter, D.M., and Will, Y. (2014). Human drug-induced liver injury severity is highly associated with dual inhibition of liver mitochondrial function and bile salt export pump. Hepatology 60, 1015-1022.
Asai, A., Aihara, E., Watson, C., Mourya, R., Mizuochi, T., Shivakumar, P., Phelan, K., Mayhew, C., Helmrath, M., Takebe, T., et al. (2017). Paracrine signals regulate human liver organoid maturation from induced pluripotent stem cells. Development 144, 1056-1064.
Barth, C.A., and Schwarz, L.R. (1982). Transcellular transport of fluorescein in hepatocyte monolayers: evidence for functional polarity of cells in culture. Proc Natl Acad Sci U S A 79, 4985-4987.
Begriche, K., Massart, J., Robin, M.A., Borgne-Sanchez, A., and Fromenty, B. (2011). Drug-induced toxicity on mitochondria and lipid metabolism: mechanistic diversity and deleterious consequences for the liver. J Hepatol 54, 773-794.
Bell, L.N., and Chalasani, N. (2009). Epidemiology of idiosyncratic drug-induced liver injury. Semin Liver Dis 29, 337-347.
Bernardi, P. (1996). The permeability transition pore. Control points of a cyclosporin A-sensitive mitochondrial channel involved in cell death. Biochim Biophys Acta 1275, 5-9.
Bohan, T.P., Helton, E., McDonald, I., Konig, S., Gazitt, S., Sugimoto, T., Scheffner, D., Cusmano, L., Li, S., and Koch, G. (2001). Effect of L-carnitine treatment for valproate-induced hepatotoxicity. Neurology 56, 1405-1409.
Bravo, P., Bender, V., and Cassio, D. (1998). Efficient in vitro vectorial transport of a fluorescent conjugated bile acid analogue by polarized hepatic hybrid WIF-B and WIF-B9 cells. Hepatology 27, 576-583.
Browning, J.D., and Horton, J.D. (2004). Molecular mediators of hepatic steatosis and liver injury. J Clin Invest 114, 147-152.
Chang, J.H., Plise, E., Cheong, J., Ho, Q., and Lin, M. (2013). Evaluating the in vitro inhibition of UGT1A1, OATP1B1, OATP1B3, MRP2, and BSEP in predicting drug-induced hyperbilirubinemia. Mol Pharm 10, 3067-3075.
Chatterjee, S., Richert, L., Augustijns, P., and Annaert, P. (2014). Hepatocyte-based in vitro model for assessment of drug-induced cholestasis. Toxicol Appl Pharmacol 274, 124-136.
Chughlay, M.F., Kramer, N., Spearman, C.W., Werfalli, M., and Cohen, K. (2016). N-acetylcysteine for non-paracetamol drug-induced liver injury: a systematic review. Br J Clin Pharmacol 81, 1021-1029.
Consortium, E.P. (2012). An integrated encyclopedia of DNA elements in the human genome. Nature 489, 57-74.
Cutrin, J.C., Cantino, D., Biasi, F., Chiarpotto, E., Salizzoni, M., Andorno, E., Massano, G., Lanfranco, G., Rizzetto, M., Boveris, A., et al. (1996). Reperfusion damage to the bile canaliculi in transplanted human liver. Hepatology 24, 1053-1057.
D'Amour, K.A., Agulnick, A.D., Eliazer, S., Kelly, O.G., Kroon, E., and Baetge, E.E. (2005). Efficient differentiation of human embryonic stem cells to definitive endoderm. Nat Biotechnol 23, 1534-1541.
Davidson, M.D., Ballinger, K.R., and Khetani, S.R. (2016). Long-term exposure to abnormal glucose levels alters drug metabolism pathways and insulin sensitivity in primary human hepatocytes. Sci Rep 6, 28178.
Dumortier, G., Cabaret, W., Stamatiadis, L., Saba, G., Benadhira, R., Rocamora, J.F., Aubriot-Delmas, B., Glikman, J., and Januel, D. (2002). [Hepatic tolerance of atypical antipsychotic drugs]. Encephale 28, 542-551.
Dvir-Ginzberg, M., Gamlieli-Bonshtein, I., Agbaria, R., and Cohen, S. (2003). Liver tissue engineering within alginate scaffolds: effects of cell-seeding density on hepatocyte viability, morphology, and function. Tissue Eng 9, 757-766.
Edling, Y., Sivertsson, L.K., Butura, A., Ingelman-Sundberg, M., and Ek, M. (2009). Increased sensitivity for troglitazone-induced cytotoxicity using a human in vitro co-culture model. Toxicol In Vitro 23, 1387-1395.
Fahrmayr, C., Konig, J., Auge, D., Mieth, M., Munch, K., Segrestaa, J., Pfeifer, T., Treiber, A., and Fromm, M. (2013). Phase I and II metabolism and MRP2-mediated export of bosentan in a MDCKII-OATP1B1-CYP3A4-UGT1A1-MRP2 quadruple-transfected cell line. Br J Pharmacol 169, 21-33.
Falasca, L., Favale, A., Serafino, A., Ara, C., and Conti Devirgiliis, L. (1998). The effect of retinoic acid on the re-establishment of differentiated hepatocyte phenotype in primary culture. Cell Tissue Res 293, 337-347.
Fisher, A., Croft-Baker, J., Davis, M., Purcell, P., and McLean, A.J. (2002). Entacapone-induced hepatotoxicity and hepatic dysfunction. Mov Disord 17, 1362-1365; discussion 1397-1400.
Fromenty, B. (2013). Drug-induced liver injury in obesity. J Hepatol 58, 824-826.
Heidari, R., Niknahad, H., Jamshidzadeh, A., and Abdoli, N. (2014). Factors affecting drug-induced liver injury: antithyroid drugs as instances. Clin Mol Hepatol 20, 237-248.
Inoue, H., Nagata, N., Kurokawa, H., and Yamanaka, S. (2014). iPS cells: a game changer for future medicine. EMBO J 33, 409-417.
Jalan-Sakrikar, N., De Assuncao, T.M., Lu, J., Almada, L.L., Lomberk, G., Fernandez-Zapico, M.E., Urrutia, R., and Huebert, R.C. (2016). Hedgehog Signaling Overcomes an EZH2-Dependent Epigenetic Barrier to Promote Cholangiocyte Expansion. PLoS One 11, e0168266.
Kock, K., and Brouwer, K.L. (2012). A perspective on efflux transport proteins in the liver. Clin Pharmacol Ther 92, 599-612.
Krahenbuhl, S., Talos, C., Fischer, S., and Reichen, J. (1994). Toxicity of bile acids on the electron transport chain of isolated rat liver mitochondria. Hepatology 19, 471-479.
Le Vee, M., Noel, G., Jouan, E., Stieger, B., and Fardel, O. (2013). Polarized expression of drug transporters in differentiated human hepatoma HepaRG cells. Toxicol In Vitro 27, 1979-1986.
Lechner, C., Reichel, V., Moenning, U., Reichel, A., and Fricker, G. (2010). Development of a fluorescence-based assay for drug interactions with human Multidrug Resistance Related Protein (MRP2; ABCC2) in MDCKII-MRP2 membrane vesicles. Eur J Pharm Biopharm 75, 284-290.
Lee, W.M., Hynan, L.S., Rossaro, L., Fontana, R.J., Stravitz, R.T., Larson, A.M., Davern, T.J., 2nd, Murray, N.G., McCashland, T., Reisch, J.S., et al. (2009). Intravenous N-acetylcysteine improves transplant-free survival in early stage non-acetaminophen acute liver failure. Gastroenterology 137, 856-864, 864 e851.
Leslie, E.M., Watkins, P.B., Kim, R.B., and Brouwer, K.L. (2007). Differential inhibition of rat and human Na+-dependent taurocholate cotransporting polypeptide (NTCP/SLC10A1)by bosentan: a mechanism for species differences in hepatotoxicity. J Pharmacol Exp Ther 321, 1170-1178.
Li, N., Oquendo, E., Capaldi, R.A., Robinson, J.P., He, Y.D., Hamadeh, H.K., Afshari, C.A., Lightfoot-Dunn, R., and Narayanan, P.K. (2014). A systematic assessment of mitochondrial function identified novel signatures for drug-induced mitochondrial disruption in cells. Toxicol Sci 142, 261-273.
Makin, A.J., Wendon, J., and Williams, R. (1995). A 7-year experience of severe acetaminophen-induced hepatotoxicity (1987-1993). Gastroenterology 109, 1907-1916.
Malinen, M.M., Kanninen, L.K., Corlu, A., Isoniemi, H.M., Lou, Y.R., Yliperttula, M.L., and Urtti, A.O. (2014). Differentiation of liver progenitor cell line to functional organotypic cultures in 3D nanofibrillar cellulose and hyaluronan-gelatin hydrogels. Biomaterials 35, 5110-5121.
Marcum, Z.A., and Gellad, W.F. (2012). Medication adherence to multidrug regimens. Clin Geriatr Med 28, 287-300.
Michaut, A., Le Guillou, D., Moreau, C., Bucher, S., McGill, M.R., Martinais, S., Gicquel, T., Morel, I., Robin, M.A., Jaeschke, H., et al. (2016). A cellular model to study drug-induced liver injury in nonalcoholic fatty liver disease: Application to acetaminophen. Toxicol Appl Pharmacol 292, 40-55.
Miki, T., Ring, A., and Gerlach, J. (2011). Hepatic differentiation of human embryonic stem cells is promoted by three-dimensional dynamic perfusion culture conditions. Tissue Eng Part C Methods 17, 557-568.
Mork, L.M., Isaksson, B., Boran, N., Ericzon, B.G., Strom, S., Fischler, B., and Ellis, E. (2012). Comparison of culture media for bile Acid transport studies in primary human hepatocytes. J Clin Exp Hepatol 2, 315-322.
Navarro, V.J., and Senior, J.R. (2006). Drug-related hepatotoxicity. N Engl J Med 354, 731-739.
Ni, X., Gao, Y., Wu, Z., Ma, L., Chen, C., Wang, L., Lin, Y., Hui, L., and Pan, G. (2016). Functional human induced hepatocytes (hiHeps) with bile acid synthesis and transport capacities: A novel in vitro cholestatic model. Sci Rep 6, 38694.
Nishida, T., Gatmaitan, Z., Che, M., and Arias, I.M. (1991). Rat liver canalicular membrane vesicles contain an ATP-dependent bile acid transport system. Proc Natl Acad Sci U S A 88, 6590-6594.
Oorts, M., Baze, A., Bachellier, P., Heyd, B., Zacharias, T., Annaert, P., and Richert, L. (2016). Drug-induced cholestasis risk assessment in sandwich-cultured human hepatocytes. Toxicol In Vitro 34, 179-186.
Pessayre, D., Fromenty, B., Berson, A., Robin, M.A., Letteron, P., Moreau, R., and Mansouri, A. (2012). Central role of mitochondria in drug-induced liver injury. Drug Metab Rev 44, 34-87.
Pessayre, D., Mansouri, A., Berson, A., and Fromenty, B. (2010). Mitochondrial involvement in drug-induced liver injury. Handb Exp Pharmacol, 311-365.
Polson, J., and Lee, W.M. (2005). AASLD position paper: the management of acute liver failure. Hepatology 41, 1179-1197.
Rachek, L.I., Yuzefovych, L.V., Ledoux, S.P., Julie, N.L., and Wilson, G.L. (2009). Troglitazone, but not rosiglitazone, damages mitochondrial DNA and induces mitochondrial dysfunction and cell death in human hepatocytes. Toxicol Appl Pharmacol 240, 348-354.
Rector, R.S., Thyfault, J.P., Uptergrove, G.M., Morris, E.M., Naples, S.P., Borengasser, S.J., Mikus, C.R., Laye, M.J., Laughlin, M.H., Booth, F.W., et al. (2010). Mitochondrial dysfunction precedes insulin resistance and hepatic steatosis and contributes to the natural history of non-alcoholic fatty liver disease in an obese rodent model. J Hepatol 52, 727-736.
Russo, M.W., Galanko, J.A., Shrestha, R., Fried, M.W., and Watkins, P. (2004). Liver transplantation for acute liver failure from drug induced liver injury in the United States. Liver Transpl 10, 1018-1023.
Sébastien Lê, J.J., François Husson (2008). FactoMineR: An R Package for Multivariate Analysis. Journal of Statistical Software 25.
Serviddio, G., Pereda, J., Pallardo, F.V., Carretero, J., Borras, C., Cutrin, J., Vendemiale, G., Poli, G., Vina, J., and Sastre, J. (2004). Ursodeoxycholic acid protects against secondary biliary cirrhosis in rats by preventing mitochondrial oxidative stress. Hepatology 39, 711-720.
Sloan, C.A., Chan, E.T., Davidson, J.M., Malladi, V.S., Strattan, J.S., Hitz, B.C., Gabdank, I., Narayanan, A.K., Ho, M., Lee, B.T., et al. (2016). ENCODE data at the ENCODE portal. Nucleic Acids Res 44, D726-732.
Song, W., Lu, Y.C., Frankel, A.S., An, D., Schwartz, R.E., and Ma, M. (2015). Engraftment of human induced pluripotent stem cell-derived hepatocytes in immunocompetent mice via 3D co-aggregation and encapsulation. Sci Rep 5, 16884.
Song, Z., Cai, J., Liu, Y., Zhao, D., Yong, J., Duo, S., Song, X., Guo, Y., Zhao, Y., Qin, H., et al. (2009). Efficient generation of hepatocyte-like cells from human induced pluripotent stem cells. Cell Res 19, 1233-1242.
Spence, J.R., Mayhew, C.N., Rankin, S.A., Kuhar, M.F., Vallance, J.E., Tolle, K., Hoskins, E.E., Kalinichenko, V.V., Wells, S.I., Zorn, A.M., et al. (2011). Directed differentiation of human pluripotent stem cells into intestinal tissue in vitro. Nature 470, 105-109.
Stevens, J.L., and Baker, T.K. (2009). The future of drug safety testing: expanding the view and narrowing the focus. Drug Discov Today 14, 162-167.
Takahashi, K., Tanabe, K., Ohnuki, M., Narita, M., Ichisaka, T., Tomoda, K., and Yamanaka, S. (2007). Induction of pluripotent stem cells from adult human fibroblasts by defined factors. Cell 131, 861-872.
Takebe, T., and Taniguchi, H. (2014). Human iPSC-derived miniature organs: a tool for drug studies. Clin Pharmacol Ther 96, 310-313.
Tian, X., Zamek-Gliszczynski, M.J., Zhang, P., and Brouwer, K.L. (2004). Modulation of multidrug resistance-associated protein 2 (Mrp2) and Mrp3 expression and function with small interfering RNA in sandwich-cultured rat hepatocytes. Mol Pharmacol 66, 1004-1010.
Tsukada, N., Ackerley, C.A., and Phillips, M.J. (1995). The structure and organization of the bile canalicular cytoskeleton with special reference to actin and actin-binding proteins. Hepatology 21, 1106-1113.
Verma, S., and Kaplowitz, N. (2009). Diagnosis, management and prevention of drug-induced liver injury. Gut 58, 1555-1564.
Vosough, M., Omidinia, E., Kadivar, M., Shokrgozar, M.A., Pournasr, B., Aghdami, N., and Baharvand, H. (2013). Generation of functional hepatocyte-like cells from human pluripotent stem cells in a scalable suspension culture. Stem Cells Dev 22, 2693-2705.
Yang, K., Woodhead, J.L., Watkins, P.B., Howell, B.A., and Brouwer, K.L. (2014). Systems pharmacology modeling predicts delayed presentation and species differences in bile acid-mediated troglitazone hepatotoxicity. Clin Pharmacol Ther 96, 589-598.
Zborowski, J., and Wojtczak, L. (1963). Induction of Swelling of Liver Mitochondria by Fatty Acids of Various Chain Length. Biochim Biophys Acta 70, 596-598.

All percentages and ratios are calculated by weight unless otherwise indicated.

All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "20 mm" is intended to mean "about 20 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.
The invention is also illustrated by the following numbered paragraphs:
1. A method of inducing formation of a liver organoid from iPSC cells, comprising the steps of:
   a) contacting definitive endoderm (DE) derived from said iPSC cells with a FGF pathway activator and a GSK3 inhibitor, for a period of time sufficient to form posterior foregut spheroids, preferably for a period of time of from about 1 day to about 3 days;
   b) incubating said posterior foregut spheroids of step a in the presence of retinoic acid (RA) for a period of time sufficient to form said liver organoid, preferably for a period of time of from about 1 to about 5 days, preferably about 4 days.
2. The method of paragraph 1, wherein said stem cells are human iPSCs.
3. The method of paragraphs 1 or 2, wherein said foregut spheroids are embedded in a basement membrane matrix, preferably Matrigel.
4. The method of any preceding paragraphs, wherein said HLOs are characterized in that said HLOs express alpha-fetoprotein (AFP), albumin (ALB), retinal binding protein (RBP4), cytokeratin 19 (CK19), hepatocyte nuclear factor 6 (HNF6), and cytochrome P450 3A4 (CYP3A4), HNF4a, E-cadherin, DAPI, and Epcam, preferably when expression is measured at day 40 to day 50.
5. The method of any preceding paragraphs, wherein said HLOs are characterized in that said HLOs have bile transport activity.
6. A liver organoid derived from a stem cell, comprising a luminal structure comprising internalized microvilli comprising mesenchymal cells wherein said luminal structure is surrounded by polarized hepatocytes, and basement membrane.
7. The liver organoid of paragraphs 6 wherein said stem cell is a human iPSC.
8. The liver organoid of paragraphs 6 or paragraphs 7 wherein said liver organoid comprises functional stellate cells and functional Kupffer cells.
9. The liver organoid of any of paragraphs 6 through 8 wherein said liver organoid is characterized by having one or more of the following: bile production capacity, bile transport activity, Complement factor H expression of at least 50 ng/mL/1xe⁶ cells/24hr, Complement factor B of at least 40 ng/mL/1xe⁶ cells/24hr, C3 expression of at least 1000 ng/mL/1xe⁶ cells/24hr; C4 expression of at least 1000 ng/mL/1xe⁶ cells/24hr, fibrinogen production of at least 1,000 ng/mL/1xe⁶ cells/24hr and albumin production of at least 1,000 ng/mL/1xe⁶ cells/24hr.
10. The liver organoid of any of paragraphs 6 through 9 wherein said liver organoid is characterized by having total hepatic protein expression of at least 10,000 ng/mL 1xe⁶ cells/24 hours.
11. The liver organoid of any of paragraphs 6 through 10 wherein said liver organoid expresses one or more genes selected from PROX1, RBP4, CYP2C9, CYP3A4, ABCC11, CFH, C3, C5, ALB, FBG, MRP2, ALCAM, CD68, CD34, CD31.
12. The liver organoid of any of paragraphs 6 through 11 wherein said HLO comprises a drug metabolism cytochrome variant, preferably a CY2C9*2 variant.
13. The liver organoid of any of paragraphs 6 through 12, wherein said liver organoid is free does not comprise inflammatory cells, for example T-cells or other inflammatory secreted proteins.
14. A method of screening for a serious adverse event (SAE), preferably liver failure and/or drug induced liver injury (DILI), comprising the step of contacting a drug of interest with the liver organoid of any preceding paragraphs.
15. The method of paragraphs 14 wherein said method comprises the step of measuring intake and/or efflux of fluorescein diacetate (FD), wherein impaired efflux indicates that said drug is likely to induce a serious adverse event.
16. The method of paragraphs 14 or 15, wherein the toxicity of said drug of interest is determined by measurement of a parameter selected from mitochondria membrane potential, measurement of ROS, swelling of liver mitochondria, and combinations thereof, wherein injury to said mitochondria indicates that said drug is likely to induce a serious adverse event.
17. The method of any of paragraphs 14 through 16, wherein said method comprises the step of assaying organoid viability, wherein an impaired organoid viability determination indicates that said drug is likely to induce a serious adverse event.
18. A method of treating an individual having liver damage, comprising implanting a liver organoid according to any preceding paragraphs into said individual.
19. The method of paragraphs 18, wherein said liver damage is selected from metabolic liver disease, end stage liver disease, or a combination thereof.
20. A method of identifying a preferred therapeutic agent for an individual, comprising contacting a liver organoid derived from an iPSC of interest with a candidate compound.
21. The method of paragraphs 20, wherein said iPSC of interest comprises one or more mutations found in said individual.
22. The method of paragraphs 20 or 21, wherein said iPSC of interest is derived from the same ethic background of said individual.
23. The method of any of paragraphs 20 through 22, wherein said iPSC of interest is derived from said individual.

## Claims

1. A liver organoid derived from a stem cell, comprising: a luminal structure comprising internalized microvilli and mesenchymal cells, wherein said luminal structure is surrounded by polarized hepatocytes; and basement membrane.

2. The liver organoid of claim 1 wherein said stem cell is a human iPSC.

3. The liver organoid of claim 1 or claim 2 wherein said liver organoid comprises functional stellate cells and functional Kupffer cells.

4. The liver organoid of any one of claims 1 to 3, wherein:
a) said liver organoid is **characterized by** having one or more of the following: bile production capacity, bile transport activity, Complement factor H expression of at least 50 ng/mL/1xe⁶ cells/24hr, Complement factor B of at least 40 ng/mL/1xe⁶ cells/24hr, C3 expression of at least 1000 ng/mL/1xe⁶ cells/24hr; C4 expression of at least 1000 ng/mL/1xe⁶ cells/24hr, fibrinogen production of at least 1,000 ng/mL/Ixe6 cells/24hr and albumin production of at least 1,000 ng/mL/1xe⁶ cells/24hr; and/or
b) said liver organoid is **characterized by** having total hepatic protein expression of at least 10,000 ng/mL/1xe⁶ cells/24 hours.

5. The liver organoid of any one of claims 1 to 4 wherein said liver organoid expresses one or more genes selected from PROX1, RBP4, CYP2C9, CYP3A4, ABCC11, CFH, C3, *CS,* ALB, FBG, MRP2, ALCAM, CD68, CD34, CD31.

6. The liver organoid of any one of claims 1 to 5 wherein said HLO comprises a drug metabolism cytochrome variant, preferably a CY2C9*2 variant.

7. The liver organoid of any one of claims 1 to 6, wherein said liver organoid does not comprise inflammatory cells, for example T-cells or other inflammatory secreted proteins.

8. The liver organoid of any one of claims 1-7, wherein said liver organoid is formed by a method comprising the steps of:
a) contacting definitive endoderm (DE) derived form iPSC cells with a FGF pathway activator and a GSK3 inhibitor, for a period of time sufficient to form posterior foregut spheroids, preferably for a period of time of from about 1 day to about 3 days;
b) incubating said posterior foregut spheroids of step a in the presence of retinoic acid (RA) for a period of time sufficient to form said liver organoid, preferably for a period of time of from about 1 to about 5 days, preferably about 4 days;
optionally wherein:
i) said stem cells are human iPSCs; and/or
ii) said foregut spheroids are embedded in a basement membrane matrix, preferably Matrigel.

9. The liver organoid of claim 8, wherein:
a) said liver organoids are **characterized in that** the liver organoids express alpha-fetoprotein (AFP), albumin (ALB), retinol binding protein (RBP4), cytokeratin 19 (CK19), hepatocyte nuclear factor 6 (HNF6), cytochrome P450 3A4 (CYP3A4), HNFa, E-cadherin, DAPI, and/or Epcam, preferably when expression is measured at day 40 to day 50; and/or
b) said liver organoids are **characterized in that** said liver organoids have bile transport activity.

10. A method of screening for a serious adverse event (SAE), preferably liver failure and/or drug induced liver injury (DILI), comprising the step of contacting a drug of interest with the liver organoid of any preceding claim.

11. The method of claim 10 wherein the method comprises:
a) the step of measuring intake and/or efflux of fluorescein diacetate (FD), wherein impaired efflux indicates that said drug is likely to induce a serious adverse event; and/or
b) the step of assaying organoid viability, wherein an impaired organoid viability determination indicates that said drug is likely to induce a serious adverse event; and/or
c) the toxicity of said drug of interest is determined by measurement of a parameter selected from mitochondria membrane potential, measurement of ROS, swelling of liver mitochondria, and combinations thereof, wherein injury to said mitochondria indicates that said drug is likely to induce a serious adverse event.

12. The liver organoid of any one of claims 1-9 for use in a method of treating an individual having liver damage.

13. The liver organoid for use of claim 12, wherein said liver damage is selected from metabolic liver disease, end stage liver disease, or a combination thereof.

14. A method of identifying a preferred therapeutic agent for an individual, comprising contacting a liver organoid derived from an iPSC of interest with a candidate compound.

15. The method of claim 14, wherein said iPSC of interest:
a) comprises one or more mutations found in said individual;
b) is derived from the same ethic background of said individual;
c) is derived from said individual.
